# NEW EUROPEAN PATENT SPECIFICATION

(11) **EP 0 205 475 B2**
(45) Date of publication and mention of the opposition decision: **04.09.2002**
(45) Mention of the grant of the patent: 06.07.1994
(21) Application number: 85905953.7
(22) Date of filing: 04.12.1985
(51) Int. Cl.: C12P 21/00, C12N 15/15

(54) **RECOMBINANT METHODS FOR PRODUCTION OF SERINE PROTEASE INHIBITORS AND DNA SEQUENCES USEFUL FOR SAME**
REKOMBINANTVERFAHREN ZUR HERSTELLUNG VON SERINPROTEASEINHIBITOREN, SOWIE DNS-SEQUENZEN DAZU
PROCEDES DE PRODUCTION PAR RECOMBINAISON D'INHIBITEURS DE PROTEASE DE SERINE; SEQUENCES D'ADN UTILES A CES PROCEDES

(30) Priority: 06.12.1984 US 678822; 02.12.1985 US 803471
(43) Date of publication of application: 30.12.1986
(73) Proprietor: Amgen Inc.,, Thousand Oaks, California 91320-1799 (US)
(72) Inventor: BANDYOPADHYAY, Pradip, K., Boulder, CO 80302 (US); EISENBERG, Stephen, P., Boulder, CO 80302 (US); STETLER, Gary, L., Boulder, CO 80302 (US); THOMPSON, Robert, C., Boulder, CO 80303 (US)
(74) Representative: Grünecker, Kinkeldey, Stockmair & Schwanhäusser Anwaltssozietät
(86) International application number: US8502385
(87) International publication number: WO86003519

(56) References cited:
- EP-A- 103 409
- EP-A- 0 114 506
- WO-A-84/03711
- DE-A- 247 922
- Seemüller et al. FEBS Lett. (1986), Vol. 199 (1) pp.43-48
- Schiessler et al. (1978), pp. 195-207 of Neutral Proteases of Human Polymorphonuclear leucocytes, Urban and Schwarzenberg, Inc. Baltimore-Munich
- Betz, (1986), Gene 42 pp. 283-292
- Fritz et al.,(1978), Agents and Actions, Vol.8/1-2 pp. 57-64
- Ohlsson et al. in Hoppe-Seyler's Z. Physiol. Chem. Vol. 364, pp. 1323-1328, Sept. 1983
- Proc. Natl. Acad. Sci. USA, vol. 80, March 1983, pp. 1194-1198; R.A. Young et al.
- Biological Abstracts, vol. 78, 1984, ref. no. 90008, M. Ohlsson et al.
- Drug Development Research, vol. 1, 1981, pp. 435-454, Alan R. Liss, Inc.; W.L. Miller et al.
- Nature, vol. 297, June 24, 1982, pp. 655-659, Macmillan Journals Ltd.; M. Leicht et al.
- Proc. Natl. Acad. Sci. USA, vol. 80, no. 22, Nov. 1983, pp. 6838-6842, Washington, US; S. Anderson et al.
- EMBO Journal, vol. 3, no. 10, 1984, pp. 2437-2442, IRL Press Ltd, Oxford, GB; J. Ghrayeb et al.
- Gene, vol. 25, no. 2/3, Nov. 1983, ref. no. 864, pp. 167-178, Elsevier Sc. Publ., Brussels, BE; E. Amann et al.
- Angew. Chem. Int. Ed. Engl, vol. 22, 1983, pp. 842-858, Verlag Chemie GmbH, Weinheim, DE; F. Wengenmayer
- Chemical Abstracts, vol. 99: 173443u, 1983; Ohlsson, K. et al.
- Chemical Abstracts, vol. 90: 117084e, 1979; Schiessler et al.
- Chemical Abstracts, vol. 88: 132417t, 1978; Fritz, H. et al.
- Chemical Abstracts, vol. 89: 192866u, 1978; Schiessler et al.
- Biochem. Biophys. Res. Comm., vol. 116, pp. 375-382, 1983; Rogers, J. et al.
- Proc. Natl. Acad. Sci., vol. 78, pp. 6826-6830, 1981; Kurachi, K. et al.
- Proc. Natl. Acad. Sci., vol. 81, pp. 5403-5407, 1984; Schoner, B. et al.
- Ann. Rev. Biochem., vol. 52, pp. 655-709, 1983; Travis, J. et al.

## Description

### BACKGROUND OF THE INVENTION

Endogenous proteolytic enzymes serve to degrade invading organisms, antigen-antibody complexes and certain tissue proteins which are no longer necessary or useful to the organism. In a normally functioning organism, proteolytic enzymes are produced in a limited quantity and are regulated in part through the synthesis of protease inhibitors.

A large number of naturally-occurring protease inhibitors serve to control the endogenous proteases by limiting their reactions locally and temporally. In addition, the protease inhibitors may inhibit proteases introduced into the body by infective and parasitic agents. Tissues that are particularly prone to proteolytic attack and infection, e.g., those of the respiratory tract, are rich in protease inhibitors.

Protease inhibitors comprise approximately 10% of the human plasma proteins. At least eight inhibitors have been isolated from this source and characterized in the literature. These include α₂-macroglobulin (α₂M), α₁-protease inhibitor (α₁PI), α₁-antichymotrypsin (α₁Achy), β₁-anticollagenase (β₁AC), and inter-α-trypsin inhibitor (IαI).

A disturbance of the protease/protease inhibitor balance can lead to protease mediated tissue destruction, including emphysema, arthritis, glomerulonephritis, periodontitis, muscular dystrophy, tumor invasion and various other pathological conditions. In certain situations, e.g., severe pathological processes such as sepsis or acute leukemia, the amount of free proteolytic enzymes present increases due to the release of enzyme from the secretory cells. In addition, or separately in other situations, a diminished regulating inhibitor capacity of the organism may also cause alterations in the protease/protease inhibitor balance. An example of such a diminished regulating inhibitor capacity is α₁-protease inhibitor deficiency, which is highly correlated with the development of pulmonary emphysema.

In organisms where such aberrant conditions are present, serious damage to the organism can occur unless measures can be taken to control the proteolytic enzymes. Therefore, protease inhibitors have been sought which are capable of being administered to an organism to control the proteolytic enzymes.

Leukocyte elastase is an example of a serine protease of particular interest from a phamacological standpoint. Leukocyte elastase, when released extracellularly, degrades connective tissue and other valuable proteins. While it is necessary for a normally functioning organism to degrade a certain amount of connective tissue and other proteins, the presence of an excessive amount of leukocyte elastase has been associated with various pathological states, such as emphysema and rheumatoid arthritis. To counteract the effects of leukocyte elastase when it is present in amounts greater than normal, a protease inhibitor has been sought which is effective against leukocyte elastase. Such a protease inhibitor would be especially useful if it were capable of being prepared, via a recombinant DNA method, in a purified form and in sufficient quantities to be pharmaceutically useful.

In the past, at least two leukocyte elastase inhibitors have been identified in the literature. One protein, described in Schiessler et al., "Acid-Stable Inhibitors of Granulocyte Neutral Proteases in Human Mucous Secretions: Biochemistry and Possible Biological Function," in Neutral Proteases of Human Polymor-phoneuclear Leucocytes, Havemann et al. (eds), Urban and Schwarzenberg, Inc. (1978), was isolated from human seminal plasma and sputum and was characterized as being approximately 11 Kda in size with tyrosine as the N-terminal amino acid. The literature reports of this protein have only furnished a partial amino acid sequence, but even this partial sequence indicates that this protein varies substantially from the proteins of the present invention. The reports of the sequence of this protein, in combination with amino acid sequence data for proteins of the present invention, indicate to the present inventors that the product sequenced by Schiessler et al. may have been a degraded protein which was not a single-polypeptide chain.

A second protein, isolated in one instance from human plasma, has been named α₁-protease inhibitor. Work on this protein has been summarized in a review by Travis and Salvesen, Annual Review of Biochemistry 52: 655-709 (1983). The reports of the amino acid sequence of this protein indicate that it too differs substantially from the proteins of the present invention.

Because of the substantial differences in structure between single-polypeptide-chain proteins of the present invention and any single-polypeptide-chain serine protease inhibitors of the prior art, the single-polypeptide-chain serine protease inhibitors of the prior art are not "substantially homologous" to the proteins of the present invention.

Trypsin is another protease of particular interest from a pharmacological standpoint. Trypsin is known to initiate degradation of certain soft organ tissue, such as pancreatic tissue, during a variety of acute conditions, such as pancreatitis. Various efforts have been directed toward the treatment of these conditions, without marked success, through the use of proteins which it was hoped would inhibit the action of trypsin. Illustrative of such efforts are attempts to use exogenous bovine trypsin inhibitors in treatment of human pancreatitis. While such techniques have been attempted in Europe, they have not been approved as effective by the U.S. Food and Drug Administration. Thus, there is a need for a protease inhibitor effective in neutralizing excess trypsin in a variety of acute and chronic conditions. As was the case with the leukocyte elastase inhibitor discussed above, a trypsin inhibitor would be particularly useful if it could be isolated and prepared, by recombinant DNA methods, in a purified form and in sufficient quantities to be pharmaceutically useful.

Cathepsin G is another protease present in large quantities in leukocytes. Cathepsin G is known to be capable of degrading in vitro a variety of valuable proteins, including those of the complement pathway. Pancreatic elastase is another protease which may have a role in pancreatitis. Thus, inhibitors for these proteases are also of potential pharmaceutical value.

Leukocyte elastase, trypsin, cathepsin G and pancreatic elastase are examples of a class of proteases known as serine proteases, which have elements of common structure and mechanism. Their activity against different substrates and their sensitivity to different inhibitors are believed to result from changes in only a few amino acid residues. By analogy, it is possible to conceive of a class of serine protease inhibitors, also having common elements of structure and mechanism, in which changes in a relatively few amino acids will result in inhibition of different proteases, and that at least one member of this class will inhibit every serine protease of the former class. The class of serine protease inhibitors would then be of substantial value.

Surprisingly, the present inventors have discovered a DNA sequence capable of directing synthesis of such a serine protease inhibitor, which inhibitor is biologically equivalent to one isolated from parotid secretions. The protease inhibitor of the present invention, prepared by the recombinant DNA methods set forth herein, is believed to have at least two active sites; one site which exhibits leukocyte elastase inhibiting properties and a second site which exhibits inhibitory activity against trypsin.

The recombinant inhibitor produced by the present invention is believed to be remarkably resistant to denaturation by heat and acids and resistant to proteolytic degradation by a variety of proteolytic enzymes. As used in this application, it is intended that "recombinant inhibitor" refer to a protease inhibitor which is produced by recombinant DNA methodology and techniques. Furthermore, the active form of the recombinant inhibitor of the present invention is thermodynamically stable under conditions that are normally encountered extracellularly in the mammalian body. Denatured forms of the recombinant protease inhibitor also have the ability to form the disulfide bonds and to form the non-covalent interactions necessary to assume an active tertiary structure in the absence of biochemical stimulus.

The DNA sequences of the present invention, set forth more fully hereinbelow, are capable of directing synthesis of a protein which differs greatly from other published leukocyte elastase inhibitor sequences. Thus, the identification of the DNA sequence of the present invention has made possible the invention of recombinant DNA methods of manufacturing the novel recombinant protease inhibitors disclosed herein.

Such recombinant methods will allow manufacture of the inhibitors in quantities and purities sufficient to provide economical pharmaceutical compositions which possess serine protease inhibitory activity. Moreover, the identification of the DNA sequence has made possible the invention of recombinant DNA methods of manufacturing analogs of the above described serine protease inhibitor.

### SUMMARY OF THE INVENTION

This invention relates to recombinant DNA methods for the manufacture of protease inhibitors generally and, more specifically, to the manufacture of recombinant inhibitors directed to human polymorphonuclear (PMN)-granulocyte proteases. In particular, this invention relates to recombinant DNA methods for the manufacture of inhibitors for human serine proteases, including leukoctye elastase and trypsin.

Additionally, the present invention relates to recombinant DNA methods for the manufacture of analogs of the instant serine protease inhibitors. The present invention also relates to synthetic and natural DNA sequences useful in the recombinant DNA methods as set forth below.

It is an object of the present invention to provide a method for recombinant DNA synthesis of a serine protease inhibitor, which inhibitor is a single polypeptide chain that exhibits serine protease inhibitor activity. These inhibitors possess activity which is biologically equivalent to that activity exhibited by native leukocyte elastase or trypsin inhibitors isolated from human parotid secretions.

To facilitate alternative recombinant DNA syntheses of these serine protease inhibitors, it is a further object of this invention to provide synthetic DNA sequences capable of directing production of these recombinant protease inhibitors, as well as equivalent natural DNA sequences. Such natural DNA sequences may be isolated from a cDNA or genomic library from which the gene capable of directing synthesis of the protease inhibitor may be identified and isolated.

Moreover, it is an object of the present invention to provide recombinant DNA methods for the manufacture of analogs of the protease inhibitors discussed above and corresponding analogous DNA sequences useful in such methods.

Additional objects and advantages of the invention will be set forth in part in the description which follows, and in part will be obvious from the description or may be learned from practice of the invention. The objects and advantages may be realized and attained by means of the instrumentalities and combinations particularly pointed out in the appended claims.

To achieve the objects and in accordance with the purposes of the present invention, a DNA sequence has been discovered which is capable of directing the production, by recombinant DNA methodology, of protease inhibitors which, in their active forms, are single-polypeptide-chain proteins that exhibit serine protease inhibitor activity. These recombinant protease inhibitors are remarkably resistant to denaturation by heat and acids. Furthermore, these protease inhibitors retain their biological activity even after exposure to many proteolytic enzymes, such as chymotrypsin, mouse submaxillary protease and clostripain.

The coding strand of a DNA sequence which has been discovered to direct manufacture of these recombinant serine protease inhibitors is:

5' AGCGG TAAAA GCTTC AAAGC TGGCG TATGC CCGCC
GAAAA AATCC GCGCA GTGTC TGCGG TACAA AAAAC
CGGAA TGCCA GTCCG ACTGG CAGTG CCCGG GTAAA
AAACG TTGTT GCCCG GACAC CTGCG GCATC AAATG
CCTGG ATCCG GTTGA TACCC CGAAC CCGAC TCGTC
GAAAA CCGGG TAAAT GCCCG GTAAC CTATG GCCAG
TGTCT GATGC TGAAC CCGCC GAACT TCTGC GAAAT
GGACG GCCAG TGTAA ACGAG ATCTG AAATG CTGTA
TGGGT ATGTG CGGCA AATCT TGTGT TTCCC CGGTA
AAAGC ATAA 3'

The nucleotides represented by the foregoing abbreviations are set forth in the Detailed Description of the Preferred Embodiment.

The coding strand for a second, preferred DNA sequence which has been discovered to direct manufacture of these recombinant serine protease inhibitors, particularly a secretory leukocyte protease inhibitor (SLPI) of the present invention, is:

5'	TCTGG	TAAAA	GCTTC	AAAGC	TGGCG	TATGC	CCGCC
	GAAAA	AATCC	GCGCA	GTGTC	TGCGG	TACAA	AAAAC
	CGGAA	TGCCA	GTCCG	ACTGG	CAGTG	CCCGG	GTAAA
	AAACG	TTGTT	GCCCG	GACAC	CTGCG	GCATC	AAATG
	CCTGG	ATCCG	GTTGA	TACCC	CGAAC	CCGAC	TCGTC
	GAAAA	CCGGG	TAAAT	GCCCG	GTAAC	CTATG	GCCAG
	TGTCT	GATGC	TGAAC	CCGCC	GAACT	TCTGC	GAAAT
	GGACG	GCCAG	TGTAA	ACGAG	ATCTG	AAATG	CTGTA
	TGGGT	ATGTG	CGGCA	AATCT	TGTGT	TTCCC	CGGTA
	AAAGC	ATAA					3'

To further achieve the objects and in accordance with the purposes of the present invention, a recombinant DNA method is disclosed which results in microbial manufacture of the instant serine protease inhibitors using either the natural or synthetic DNA sequences referred to above. This recombinant DNA method comprises:
(a) Preparation of a DNA sequence capable of directing a host microorganism to produce a protein having serine protease inhibitor activity, preferably leukocyte elastase inhibitor activity;
(b) Cloning the DNA sequence into a vector capable of being transferred into and replicating in a host microorganism, such vector containing operational elements for the DNA sequence;
(c) Transferring the vector containing the DNA sequence and operational elements into a host microorganism capable of expressing the protease inhibiting protein;
(d) Culturing the microorganism under conditions appropriate for amplification of the vector and expression of the inhibitor;
(e) Harvesting the inhibitor; and
(f) Permitting the inhibitor to assume an active tertiary structure whereby it possesses serine protease inhibitor activity.

To facilitate identification and isolation of natural DNA sequences for use in the present invention, the present inventors have developed a human parotid tissue cDNA library. This library contains the genetic information capable of directing a cell to synthesize the serine protease inhibitors of the present invention. Other natural DNA sequences which may be used in the recombinant DNA methods set forth herein may be isolated from a human genomic library.

The synthetic DNA sequences useful in the processes of the present invention may be prepared by polynucleotide synthesis and sequencing techniques known to those of ordinary skill in the art. The natural DNA sequences useful in the foregoing process may be identified and isolated through a method comprising:
(a) Preparation of a human cDNA library from cells, preferably parotid cells, capable of generating a serine protease inhibitor;
(b) Probing the human DNA library with at least one probe capable of binding to the protease inhibitor gene or its protein product;
(c) Identification of at least one clone containing the gene coding for the inhibitor by virtue of the ability of the clone to bind at least one probe for the gene or its protein product;
(d) Isolation of the gene coding for the inhibitor from the clone(s) identified; and
(e) Linking the gene, or suitable fragments thereof, to operational elements necessary- to maintain and express the gene in a host microorganism.

The natural DNA sequences useful in the foregoing process may also be identified and isolated through a method comprising:
(a) Preparation of a human genomic DNA library, preferably propagated in a recArecBC E. coli host;
(b) Probing the human genomic DNA library with at least one probe capable of binding to a serine protein inhibitor gene or its protein product;
(c) Identification of at least one clone containing the gene coding for the inhibitor by virtue of the ability of the clone to bind at least one probe for the gene or its protein product;
(d) Isolation of the gene coding for the inhibitor from the clone or clones identified; and
(e) Linking the gene, or suitable fragments thereof, to operational elements necessary to maintain and express the gene in a host microorganism.

Moreover, to achieve the objects and in accordance with the purposes of the present invention, pharmaceutically useful analogs of the serine protease inhibitor may be produced by the above-recited recombinant DNA method by altering the synthetic DNA sequence or the natural DNA segment, through recombinant DNA techniques, to create a gene capable of inducing expression of the desired analog when cloned into an appropriate vector and transferred into an appropriate host microorganism.

Additionally, to achieve the objects and in accordance with the purposes of the present invention, pharmaceutical compositions containing, as an active ingredient, a recombinant protease inhibitor in accordance with the present invention, or its biologically active analog produced by the above-recited recombinant DNA methods, are disclosed.

The accompanying drawings, which are incorporated herein and constitute a part of this application, illustrate various plasmids useful in this invention and, together with the description, serve to explain the principles of the invention.

### BRIEF DESCRIPTION OF THE DRAWINGS

Figure 1 is a map of plasmid pSGE6.
Figure 2 is a map of plasmid pSGE8.
Figure 3 is a map of plasmid pGS285.
Figure 4 is a map of plasmid pGS485.

### DESCRIPTION OF THE PREFERRED EMBODIENTS

Reference will now be made in detail to the presently preferred embodiments of the invention, which, together with the following example, serve to explain the principles of the invention.

The present invention relates to means and processes for producing protease inhibitors which have been isolated in a purified form.

Preferred protease inhibitors produced by the present recombinant methods are described in United States Patent Application Serial No. 678,823 of Robert C. Thompson et al. entitled "Serine Protease Inhibitors and Methods for Isolation of Same," filed December 6, 1984 and United States Patent Application Serial No. 06/803,423 of Robert C. Thompson et. al. entitled "Serine Protease Inhibitors and Methods for Isolation of Same," filed of even date herewith which is issued to U.S. Patent No. 4,760,130 on July 26, 1988. Such protease inhibitors are remarkably resistant to denaturation by heat and acids and resistant to loss of activity when exposed to many proteolytic enzymes, including chymotrypsin, mouse submaxillary protease and clostripain. These inhibitors also have the ability to form the necessary disulfide bonds and undergo appropriate non-covalent interactions to assume an active tertiary structure capable of expressing serine protease inhibitor activity in the absence of a biochemical stimulus or, if the disulfide bonds have been broken and the non-covalent interactions have been disrupted, to re-form such bonds and interactions to regain such active tertiary structure in the absence of biochemical stimulus.

A preferred serine protease inhibitor having these characteristics has been sequenced. The sequence was determined to be as follows:

Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala.

The foregoing abbreviations correspond to the amino acid residues in the polypeptide as follows:

| Amino acid | Abbreviation |
|---|---|
| Alanine | Ala |
| Valine | Val |
| Leucine | Leu |
| Isoleucine | lie |
| Proline | Pro |
| Phenylalanine | Phe |
| Tryptophan | Trp |
| Methionine | Met |
| Glycine | Gly |
| Serine | Ser |
| Threonine | Thr |
| Cysteine | Cys |
| Tyrosine | Tyr |
| Asparagine | Asn |
| Glutamine | Gln |
| Aspartic acid | Asp |
| Glutamic acid | Glu |
| Lysine | Lys |
| Arginine | Arg |
| Histidine | His |

It has been found that these protease inhibitors manufactured by the recombinant DNA methods disclosed herein have more than one distinct domain. By more than one distinct domain it is meant that the protein has multiple active sites which are functional against different enzymes. The presence and location of these sites have been determined by the discovery of a substantial homology between at least two portions of the protease inhibitor. It is believed that the presence of distinct domains confers on the instant protease inhibitors the ability to inhibit a wide variety of serine proteases that includes both leukocyte elastase and trypsin.

It has further been noted that, due to the plurality of distinct domains of these protease inhibitors, the protease inhibitors may serve as frameworks on which various other active sites may be constructed to create protease inhibitors having additional properties. The preferred embodiment of the present invention involves production of a protease inhibitor that inhibits leukocyte elastase, cathepsin G, pancreatic elastase and trypsin. These enzymes are all members of a class of proteases known as serine proteases that share a common mechanism and many structural features. It is believed that, through manipulation of a few amino acid side-chains on the protease inhibitors produced by the present invention, a multiplicity of inhibitors may be created, each being capable of inhibiting at least one member of the whole class of serine proteases. Furthermore, such side-chain modifications can be expected to yield a plurality of inhibitors having improved inhibitory properties with respect to particular members of the class of serine proteases described above.

The amino acid-side chain changes required to attain these goals are suggested by certain elements of structural similarity between the preferred inhibitor produced by the present invention and other serine protease inhibitors for which the important functional part of the inhibitor has been elucidated through X-ray crystallography. Those elements of structural similarity incude amino acids 17 to 29 and amino acids 70 to 83 of the preferred serine protease inhibitor produced by the present invention described above. The changes suggested to improve the inhibitor's activity, either in terms of quantity or quality, toward trypsin-like serine proteases include changing one or more of amino acid 20 from Arg to Lys, amino acid 72 or 74 from Leu to Lys or Arg, and amino acid 73 from Met to Lys or Arg.

The changes suggested to improve the inhibitor's activity, either in terms of quantity or quality, toward chymotrypsin-like serine proteases, including cathepsin G, include changing one or more of amino acid 20 from Arg to Phe, Tyr or Trp, amino acid 72 or 74 from Leu to Phe, Tyr or Trp, and amino acid 73 from Met to Phe, Tyr, or Trp.

The changes suggested to improve the inhibitor's activity, either in terms of quantity or quality, toward pancreatic-elastase-like serine proteases include changing one or more of amino acid 20 from Arg to Ala, amino acid 72 or 74 from Leu to Ala, and amino acid 73 from Met to Ala.

It must be borne in mind in the practice of the present invention that the alteration of amino acid sequences to confer new protease inhibiting properties on the present proteins may disrupt the inhibitor's activity toward leukocyte elastase or toward trypsin. Such effects may be determined by routine experimentation following the teachings of the present invention.

Further, it is contemplated that substitution of discrete amino acids or of discrete sequences of amino acids, as set forth above, may enhance either the leukocyte elastase inhibitory properties or the trypsin inhibitory properties of the present protease inhibitors while sacrificing some activity of the unenhanced domain. Indeed, the activity of any domain within the inhibitor protein may be eliminated entirely by appropriate amino-acid substitutions, thereby creating inhibitor proteins which are specific for one or some subset of the enzymes against which the protein is normally active. For example, substitution of Gly for Arg in position 20 deactivates the trypsin inhibitory domain while substitution of Gly for Met in the 73 position or for Leu in the 72 or 74 position deactivates the leukocyte elastase inhibitory domain. The domains may also be separated into separate proteins, each of which retains the desired inhibitory functions. The present claims extend to other processes for producing such inhibitors by these means.

The present inventors have discovered a synthetic DNA sequence which is capable of directing intracellular production of the above-discussed protease inhibitors. This sequence has the following structure: 
HindIII
5'AGC GGT AAA AGC TTC AAA GCT GGC GTA TGC CCG CCG
AluI

FnuDII RsaI HpaII
AAA AAA TCC GCG CAG TGT CTG CGG TAC AAA AAA CCG
HhaI

XmaI
GAA TGC CAG TCC GAC TGG CAG TGC CCG GGT AAA AAA
HpaII

NciI

NciI
CGT TGT TGC CCG GAC ACC TGC GGC ATC AAA TGC CTG
HpaII Fnu4HI BstNI

BamHI
GAT CCG GTT GAT ACC CCG AAC CCG ACT CGT CGA AAA
HpaII TagI

NciI HpaII BalI
CCG GGT AAA TGC CCG GTA ACC TAT GGC CAG TGT CTG
HpaII NciI HaeIII

ATG CTG AAC CCG CCG AAC TTC TGC GAA ATG GAC GGC
HaeIII

BglII
CAG TGT AAA CGA GAT CTG AAA TGC TGT ATG GGT ATG
MboI

Fnu4HI NciI
TGC GGC AAA TCT TGT GTT TCC CCG GTA AAA GCA TAA 3'
HpaII
 wherein the following nucleotides are represented by the abbreviations indicated below.

| Nucleotide | Abbreviation |
|---|---|
| deoxyadenylic acid | A |
| deoxyguanylic acid | G |
| deoxycytidylic acid | C |
| thymidylic acid | T |

The present inventors have discovered a second, preferred synthetic DNA sequence which is capable of directing extracellular production of the above-discussed protease inhibitors, particularly the secretory leukocyte protease inhibitor (SLPI) referred to above. This sequence has the following structure:

HindIII
5'TCT GGT AAA AGC TTC AAA GCT GGC GTA TGC CCG CCG
AluI

FnuDII RsaI HpaII
AAA AAA TCC GCG CAG TGT CTG CGG TAC AAA AAA CCG
HhaI

XmaI
GAA TGC CAG TCC GAC TGG CAG TGC CCG GGT AAA AAA
HpaII

NciI

HpaII
CGT TGT TGC CCG GAC ACC TGC GGC ATC AAA TGC CTG
NciI Fnu4HI BstNI

BamHI
GAT CCG GTT GAT ACC CCG AAC CCG ACT CGT CGA AAA
HpaII TaqI

HpaII HpaII BalI
CCG GGT AAA TGC CCG GTA ACC TAT GGC CAG TGT CTG
NciI NciI HaeIII

ATG CTG AAC CCG CCG AAC TTC TGC GAA ATG GAC GGC
HaeIII

BglII
CAG TGT AAA CGA GAT CTG AAA TGC TGT ATG GGT ATG
MboI

Fnu4HI NciI
TGC GGC AAA TCT TGT GTT TCC CCG GTA AAA GCA TAA 3'
HpaII

Due to multiple domain structure of the instant protease inhibitors, as noted above, variations are contemplated in the synthetic DNA sequence set forth herein which will result in a DNA sequence which is capable of directing production of the serine protease inhibitor analogs as discussed above. In particular, preferred analogs of the serine protease inhibitors manufactured by recombinant DNA techniques according to the present invention have the amino acid sequence: 
R1-Gly-Lys-Ser-Phe-Lys-Ala-Gly-Val-Cys-Pro-
Pro-Lys-Lys-Ser-Ala-Gln-Cys-Leu-R2-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-R8-R3-R9-Asn-Pro-Pro-
Asn-Phe-Cys-Glu-R4-Asp-Gly-Gln-Cys-Lys-Arg-
Asp-Leu-Lys-Cys-Cys-R5-Gly-R6-Cys-Gly-Lys-
Ser-Cys-Val-Ser-Pro-Val-Lys-R7,
 wherein,
R₁ and R₇ are the same or different and are selected from the group consisting of a substituted or unsubstituted amino acid residue or derviative thereof; and
R₂, R₃, R₄, R₅, R₆, R₈ and R₉ are the same or different and are selected from the group consisting of methionine, valine, alanine, phenylalanine, tyrosine, tryptophan, lysine, glycine and arginine.

It should be noted that the DNA sequence set forth above represents a preferred embodiment of the present invention. Due to the degeneracy of the genetic code, it is to be understood that numerous choices of nucleotides may be made which will lead to a DNA sequence capable of directing production of the instant protease inhibitors or their analogs. As such, DNA sequences which are functionally equivalent to the sequence set forth above or which are functionally equivalent to sequences which would direct production of analogs of the protease inhibitor produced pursuant to the amino acid sequence set forth above, are intended to be encompassed within the present invention. As an example of the codon substitutions that are contemplated as a result of the degenerate genetic code, the following diagram represents additional DNA sequences which are intended to be included within the scope of the present invention for manufacture of the preferred amino acid sequence enumerated above. By following the example for determining equivalent DNA sequences for production of this protein, those of ordinary skill in the art will be able to determine equivalent DNA sequences for production of analogs of the preferred amino acid sequence as well. 
10
Ser Gly Lys Ser Phe Lys Ala Gly Val Cys Pro Pro Lys Lys Ser Ala
5'TCN GGN AAP TCN TTQ AAP GCN GGN GTN TGQ CCN CCN AAP AAP TCN GCN
AGQ AGQ AGQ

20 30
Gln Cys Leu Arg Tyr Lys Lys Pro Glu Cys Gln Ser Asp Trp Gln Cys
CAP TGQ CTN CGN TAQ AAP AAP CCN GAP TGQ CAP TCN GAQ TGG CAP TGQ
TTP AGP AGQ

40
Pro Gly Lys Lys Arg Cys Cys Pro Asp Thr Cys Gly Ile Lys Cys Leu
CCN GGN AAP AAP CGN TGQ TGQ CCN GAQ ACN TGQ GGN ATQ AAP TGQ CTN
AGP ATA TTP

50 60
Asp Pro Val Asp Thr Pro Asn Pro Thr Arg Arg Lys Pro Gly Lys Cys
GAQ CCN GTN GAQ ACN CCN AAQ CCN ACN CGN CGN AAP CCN GGN AAP TGQ
AGP AGP

70 80
Pro Val Thr Tyr Gly Gln Cys Leu Met Leu Asn Pro Pro Asn Phe Cys
CCN GTN ACN TAQ GGN CAP TGQ CTN ATG CTN AAQ CCN CCN AAQ TTQ TGQ
TTP TTP

90
Glu Met Asp Gly Gln Cys Lys Arg Asp Leu Lys Cys Cys Met Gly Met
GAP ATG GAQ GGN CAP TGQ AAP CGN GAQ CTN AAP TGQ TGQ ATG GGN ATG
AGP TTP

100
Cys Gly Lys Ser Cys Val Ser Pro Val Lys Ala
TGQ GGN AAP TCN TGQ GTN TCN CCN GTN AAP GCN 3'
AGQ AGQ
 In the above sequence, abbreviations used are intended to represent the nucleotides indicated below.

| Nucleotide | Abbreviation |
|---|---|
| A,G,C,T | N |
| A,G | P |
| C,T | Q |

When selecting codons for use in the synthetic DNA sequences of the present invention, including that set forth immediately above, it is preferred that the codons used to indicate a particular amino acid be those which are associated with highly expressed proteins. Examples of these preferred codons are set forth in part in Grantham, R. et al., "Codon Catalog Usage Is a Genome Strategy Modulated For Gene Expressivity" in Nucleic Acids Research 9:r43 (1981). The preferred DNA sequence of the present invention was chosen by selecting Escherichia coli sequence codons for any of the degenerate sequences.

Additionally, it is desired to select codons which facilitate the alteration of the synthetic DNA sequence to construct additional synthetic DNA sequences which are capable of directing production of analogs of the present protease inhibitors. In particular, it is preferred that nucleotide sequences are selected which, if possible, create restriction endonuclease sites at, or close to, positions in the synthetic DNA sequence into which it may be desired to insert additional codons or at which sites it may be desired to replace a codon so that analogs may be created. In the preferred embodiment of the DNA sequence of the present invention, the restriction sites are indicated below the nucleotide sequence set forth above.

Methods of creating the synthetic DNA sequences contemplated herein are generally within the ambit of routine tasks performed by one of ordinary skill in the art guided by instant disclosure. An example of a suitable method which may be used to obtain the synthetic DNA sequence disclosed herein is set forth in Matteacci, M.D. and Caruthers, M.H., J.Am.Chem.Soc. 103:3185 (1981) and Beaucage, S.L. and Caruthers, M.H., Tetrahedron Lett. 22:1859 (1981), both of which are specifically incorporated herein by reference.

In an alternate embodiment of the present invention, a DNA sequence has been isolated from a human genomic library which encodes a preferred secretory leukocyte protease inhibitor (SLPI) of the present invention. This sequence, encoding from the fourth codon and including the introns as presently known to the inventors, is as follows: 
EcoR1 . 20 . 40 . 60
GAATTCTGGTGGGGCCACACCCACTGGTGAAAGAATAAATAGTGAGGTTTGGATTGGCC
----------INTRON--------------------------------------------
. 80 . 100 . 120
ATCAGAGTCACTCCTGCCTTCACCATGAAGTCCAGCGGCCTCTTCCCCTTCCTGGTGCTG
------------------------------------------------------------
. 140 . 160 . 180
CTTGCCCTGGAACTCTGGCACTTGGGCTTGGAAGGCTCTGAAATGTAAGTTGGAGTCACT
------------------------------------------------------------
. Pstl . 220 . 240
CTGTCTAATCTGGGCTGCAGGGTCAGAGGTGGGGTCTCCTTGTGGTGTGGGTGTGTCCCC
------------------------------------------------------------
. 260 . 280 . 300
TTCTGTAGGCTCTGATCCCTCAGCTTAGTTTCGGGAGACCTCCCTGAGGGTGGAATACAT
------------------------------------------------------------
SacI 320 . 340 . 360
GTCTGGCTGAGCTCCAAGGTTTGTGTGACAGTTTGAGCTTCTGGAAATGCTTCCTCTATG
------------------------------------------------------------
. 380 . 400 . 420
CAGCCATGCTGTCAGCCCAGGTCCCACTCTCTCTCTCTCTCTCTCTCTCTCTCTCTCTCA
------------------------------------------------------------
. 440 . 460 . 480
TACTCCGCCTTCTTCTTCACCTTGCTGCGACTCTCAAATCATTAGTTTCTGACTCTGCTT
------------------------------------------------------------
. 500 . 520 . 540
CCGTTGTGTCTTTGCTTCTGCTATTTTGTCTCTGTGCTTCTCGCTTGGGATTTAGCTCTC
------------------------------------------------------------
. 560 . 580 . 600
AACTTCTCTCACACTGGTTCTATTTATCTTTGTTTACCTCTCTCCATCTCCATCACTCCC
------------------------------------------------------------
. 620 . 640 . 660
AGCCTTCCTCTCTGCCTTTGTGTAGCCTTGTTTTGCTCTTGGGTGGAGGTCTTGACTAGA
------------------------------------------------------------
 
.          680          .          700          .          720
AGCCTGCTGCCCTTTTCTTGGGTGTGAAACGTCCCCTGTCCATTTGTCTAATTTAATCAA
------------------------------------------------------------

.          740          .          760          .          780
GCCCATCAATACACCTGGAGATCAGGCAGGCATGACCTTTGGCTTTGTGGACAGCTACT
------------------------------------------------------------

.          800          .          820          .          840
GAGGTAAGGGTCTCTCCCCCTCAAAAGTGGTGCTTTGTTCAGGAGGCATGATGGGTCCTC
------------------------------------------------------------

.          860          .          880          .          900
AGTACCCAGCCTCCTCCTACCTCTTGACTTTCTCTTCAAAAGCCTTCAAAGCTGGAGTCT
--------------END OF INTRON---------------   F  K  A  G  V

.          920          .          940          .          960
GTCCTCCTAAGAAATCTGCCCAGTGCCTTAGATACAAGAAACCTGAGTGCCAGAGTGACT
C  P  P  K  K  S  A  Q  C  L  R  Y  K  K  P  E  C  Q  S  D

.          980          .          1000          .          BamH1
GGCAGTGTCCAGGGAAGAAGAGATGTTGTCCTGACACTTGTGGCATCAAATGCCTGGATC
W  Q  C  P  G  K  K  R  C  C  P  D  T  C  G  I  K  C  L  D

.          1040          .          1060          .          1080
CTGTTGACACCCCAAACCCAAGTAAGCAGGTCGGGGAACTGGGTAGAGAGATAGCCTGGG
P  V  D  T  P  N  P   -----START INTRON--------------------

.          1100          .          1120  StuI  .          1140
GACACAGCATTAGAGGGACGGAACTGGGTGATGGGTCCTGCCAGGCCTCCTTGTCAATGC
------------------------------------------------------------

.          1160          .  PvuII          .          1200
CGTAGTGAGTCACAGTGCCCTAAGAGAAGTAGCCAGCTGGTGAAGCAGCGGGCATTTAGA
------------------------------------------------------------

.          1220          .          1240          .          1260
TAGCCAGGTAGTTGGAAGCCTCCCACCTAGTCAGCACTGGGCGGCTGGCACCTGCATAAT
------------------------------------------------------------

.          1280          .          1300          .          1320
GGGGGGCCTGAAGTTCTAGGAGAGCCAGGTGCTATGTTTGGGGGCCGCCTTAGGGAGAAG
------------------------------------------------------------

.          1340          .          1360          .          1380
GTGGTGGTGATAGAGGTGGGGAGGGGATGATCCCCCCTGCTGAAGCTGGACGAGGGGCTC
------------------------------------------------------------

.          1400          .          1420  StuI  .          1440
ACTCTAAAAAGTGGGGATGGGAGGGGTTGTATAAAGTACAAGGCCTCTGACCGGTAGCCT
-------------------------------------   END OF INTRON-----

.          1460          .          1480          .          1500
CACTCTCACCCAACCCAGCAAGGAGGAAGCCTGGGAAGTGCCCAGTGACTTATGGCCAAT
-----------------   R  R  K  P  G  K  C  P  V  T  Y  G  Q
 
. 1520 . 1540 . 1560
GTTTGATGCTTAACCCCCCAATTTCTGTGAGATGGATGGCCAGTGCAAGCGTGACTTGA
C L M L N P P N F C E M D G Q C K R D L

. 1580 . 1600 . 1620
AGTGTTGCATGGGCATGTGTGGGAAATCCTGCGTTTCCCCTGTGAAAGGTAAGCAGGGGA
K C C M G M C G K S C V S P V K --START INTRON

. SacI 1640 . . 1660 . 1680
CGAGGGCACACTGAGCTCCCTCAGCCCTCTCAGCCTCAACCCTCTGGAGGCCCAGGCATA
------------------------------------------------------------

. 1700 . 1720 . 1740
TGGGCAGGGGGACTCCTGAACCCTACTCCAAGCACAGCCTCTGTCTGACTCCCTTGTCCT
------------------------------------------------------------

. 1760 . 1780 . 1800
TCAAGAGAACTGTTCTCCAGGTCTCAGGGCCAGGATTTCCATAGGAGTCGCCTGTGGCTT
------------------------------------------------------------

. 1820 . 1840 . 1860
TGATTCTATTCTAGTGTCTCTGGGTGGGGGTCCTGGGCAAGTGTCTTTCTGAGTCTAGTT
------------------------------------------------------------

. 1880 . 1900 . 1920
TCTTTATCGGTAAAATGTACATAATGAGATGAAAGTGCTCTGCAAAGACCTATGTGCACT
------------------------------------------------------------

. 1940 . 1960 . 1980
AAGAATTATTATTCAGGTGTTTCCATCATGTTTTCTGAGGTGAAATCACAAAGGATCAGT
------------------------------------------------------------

. 2000 . 2020 . 2040
GGAGTTTGAGGATTATCTAGTTCAATGCTTTGAGTTTAGAGTTTTACGTGAAAATGAGAC
------------------------------------------------------------

. 2060 . 2080 . 2100
TTGTCTCCTGACACTAAGTCTCTCTCAACTATAGCGCTATCTTGCTATTTTCTCTATCTC
------------------------------------------------------------

. 2120 . 2140 . 2160
AGAAGGATCCTTGGGCAGGAGGAAGGATGTGGATATATGATTTGGCTGGTTTCTATGCTG
------------------------------------------------------------

. 2180 . 2200 . 2220
AAGCTCTGATCTGATTTTCTCTCACAGCTTGATTCCTGCCATATCGGAGGAGGCTCTGGA
-PROBABLE------------------ STOP
END OF INTRON

. 2240 . 2260 .
GCCTGCTCTGTGTGGTCCAGGTCCTTTCCACCCTGAGCTTGGCTCCACCACTGGT
 In this sequence, the abbreviations used for the amino acid residues are the one-letter abbreviations which are commonly employed and may be found, for example, in Biochemistry by A.L. Lehninger, 2nd ed., Worth Publishers, Inc., New York, New York (1976), pg. 72.

Using this sequence and the amino acid sequence data contained herein, a synthetic DNA sequence can be constructed, that, when added to the genomic sequence above, leads to a gene which codes for the entire protease inhibitor. Alternatively, probes may be constructed using the DNA sequence set forth above and used to retrieve a DNA segment from a human genomic library which has codons for the first three amino acids.

Additionally, such probes may be used to identify a human genomic sequence which contains an appropriate leader sequence. It is contemplated that this leader sequence, or any other appropriate leader sequence, could be used in conjunction with this genomic DNA sequence in a mammalian expression system.

In another alternate embodiment of the present invention, a cDNA clone has been isolated from a parotid library which encodes a DNA sequence capable of directing intracellular production of a preferred secretory leukocyte protease inhibitor of the present invention. This clone is included on on deposit at American Type Culture Collection, Rockville, Maryland, under Accession No. .

A recombinant DNA method for the manufacture of a protease inhibitor composed of a single polypeptide chain with at least one active site possessing serine protease inhibitor activity has been disclosed. In one embodiment of the invention, the active site functions in a manner biologically equivalent to that of the native leukocyte elastase inhibitor isolated from human parotid secretions. A natural or synthetic DNA sequence may be used to direct production of the protease inhibitors. This method comprises:
(a) Preparation of a DNA sequence capable of directing a host microorganism to produce a protein having serine protease inhibitor activity;
(b) Cloning the DNA sequence into a vector capable of being transferred into and replicated in a host microorganism, such vector containing operational elements for the DNA sequence;
(c) Transferring the vector containing the synthetic DNA sequence and operational elements into a host microorganism capable of expressing the protease inhibitor;
(d) Culturing the microorganism under conditions appropriate for amplification of the vector and expression of the inhibitor;
(e) Harvesting the inhibitor; and
(f) Permitting the inhibitor to assume an active-tertiary structure whereby it possesses serine protease inhibitory activity.

Synthetic DNA sequences contemplated for use in this method have been discussed in detail above. It is further contemplated, in an alternative embodiment, that natural DNA sequences may also-be used in this method. These sequences include cDNA or genomic DNA segments. In a preferred version of this embodiment, it is contemplated that the natural DNA sequence will be obtained by a method comprising:
(a) Preparation of a human cDNA library from cells, preferably parotid cells, capable of generating a serine protease inhibitor;
(b) Probing the human DNA library with at least one probe capable of binding to the protease inhibitor gene or its protein product;
(c) Identification of at least one clone containing the gene coding for the inhibitor by virtue of the ability of the clone to bind at least one probe for the gene or its protein product;
(d) Isolation of the gene coding for the inhibitor from the clone or clones chosen;
(e) Linking the gene, or suitable fragments thereof, to operational elements necessary to maintain and express the gene in host microorganism.

The natural DNA sequences useful in the foregoing process may also be identified and isolated through a method comprising:
(a) Preparation of a human genomic DNA library, preferably propagated in a recArecBC E. coli host;
(b) Probing the human genomic DNA library with at least one probe capable of binding to a serine protein inhibitor gene or its protein product;
(c) Identification of at least one clone containing the gene coding for the inhibitor by virtue of the ability of the clone to bind at least one probe for the gene or its protein product;
(d) Isolation of the gene coding for the inhibitor from the clone(s) identified; and
(e) Linking the gene, or suitable fragments thereof, to operational elements necessary to maintain and express the gene in a host microorganism.

In isolating a natural DNA sequence suitable for use in the above-method, it is preferred to identify the two restriction sites located within and closest to the end portions of the appropriate gene or sections of the gene. The DNA segment containing the appropriate gene is then removed from the remainder of the genomic material using appropriate restriction endonucleases. After excision, the 3' and 5' ends of the DNA sequence are reconstructed to provide appropriate DNA sequences capable of coding for the N- and C-termini of the serine protease inhibitor protein and capable of fusing the DNA sequence to its operational elements.

The vectors contemplated for use in the present invention include any vectors into which a DNA sequence as discussed above can be inserted, along with any preferred or required operational elements, and which vector can then be subsequently transferred into a host microorganism and replicated in such microorganism. Preferred vectors are those whose restriction sites have been well documented and which contain the operational elements preferred or required for transcription of the DNA sequence.

The "operational elements," as discussed herein, include at least one promoter, at least one operator, at least one leader sequence, at least one Shine-Dalgarno sequence, at least one terminator codon, and any other DNA sequences necessary or preferred for appropriate transcription and subsequent translation of the vector DNA. In particular, it is contemplated that such vectors will contain at least one origin of replication recognized by the host microorganism along with at least one selectable marker and at least one promoter sequence capable of initiating transcription of the synthetic DNA sequence. It is additionally preferred that the vector, in one embodiment, contains certain DNA sequences capable of functioning as regulators, and other DNA sequences capable of coding for regulator protein. These regulators, in one embodiment, serve to prevent expression of the synthetic DNA sequence in the presence of certain environmental conditions and, in the presence of other environmental conditions, allow transcription and subsequent expression of the protein coded for by the synthetic DNA sequence. In particular, it is preferred that regulatory segments be inserted into the vector such that expression of the synthetic DNA will not occur in the absence of, for example, isopropylthio- -d-galactoside. In this situation, the transformed microorganisms containing the synthetic DNA may be grown to a desired density prior to initiation of the expression of the protease inhibitor. In this embodiment, expression of the desired protease inhibitor is induced by addition of a substance to the microbial environment capable of causing expression of the DNA sequence after the desired density has been achieved.

Additionally, it is preferred that an appropriate secretory leader sequence be present, either in the vector or at the 5' end of the synthetic DNA sequence. The leader sequence is in a position which allows the leader sequence to be immediately adjacent to the initial portion of the nucleotide sequence capable of directing expression of the protease inhibitor without any intervening translation termination signals. The presence of the leader sequence is desired in part for one or more of the following reasons: 1) the presence of the leader sequence may facilitate host processing of the initial product to the mature recombinant protease inhibitor; 2) the presence of the leader sequence may facilitate purification of the recombinant protease inhibitors, through directing the protease inhibitor out of the cell cytoplasm; 3) the presence of the leader sequence may affect the ability of the recombinant protease inhibitor to fold to its active structure through directing the protease inhibitor out of the cell cytoplasm.

In particular, the leader sequence may direct cleavage of the initial translation product by a leader peptidase to remove the leader sequence and leave a polypeptide with the prefered amino acid sequence which has the potential of serine protease inhibitory activity. In some species of host microorganisms, the presence of the appropriate leader sequence will allow transport of the completed protein into the periplasmic space, as in the case of E. coli. In the case of certain yeasts and strains of Bacilli and Pseudomonas, the appropriate leader sequence will allow transport of the protein through the cell membrane and into the extracellular medium. In this situation, the protein may be purified from extracellular protein.

Thirdly, in the case of some of the protease inhibitors prepared by the present invention, the presence of the leader sequence may be necessary to locate the completed protein in an environment where it may fold to assume its active structure, which structure possesses the appropriate elastase-inhibitor activity.

Additional operational elements include, but are not limited to, ribosome binding sites and other DNA sequences necessary for microbial expression of foreign proteins. In a preferred embodiment of the present invention, the sequence GAGGCGCAAAAA(ATG) would be used as the ribosome binding site. The operational elements as discussed herein are routinely selected by those of ordinary skill in the art in light of prior literature and the teachings contained herein. General examples of these operational elements are set forth in B. Lewin, Genes, Wiley & Sons, New York (1983), which is specifically incorporated herein by reference. The vectors as contemplated herein may be constructed in part from portions of plasmids pBR322 and/or pIQ.

In one preferred embodiment of the present invention, an additional DNA sequence is located immediately preceding the synthetic DNA sequence which codes for the protease inhibitor. The additional DNA sequence is capable of functioning as a translational coupler, i.e., it is a DNA sequence that encodes an RNA which serves to position ribosomes immediately adjacent to the ribosome binding site of the protease inhibitor RNA with which it is contiguous. In one embodiment of the present invention, the translational coupler may be derived using the DNA sequence TAACGAGGCGCAAAAAATGAAAAAGACAGCTATCGCGATCGGAGTGTAAGAAATG and methods currently known to those of ordinary skill in the art related to translational couplers. A second, preferred translational coupler has the DNA sequence

TAACGAGGCGCAAAAAATGAAAAAGACAGCTATCGCGATCAAGGAGAAATAAATG.

Upon synthesis and isolation of all necessary and desired component parts of the above-discussed vector, the vector is assembled by methods generally known to those of ordinary skill in the art. Assembly of such vectors is believed to be within the duties and tasks performed by those with ordinary skill in the art and, as such, is capable of being performed without undue experimentation. For example, similar DNA sequences have been ligated into appropriate cloning vectors, as set forth in Schoner et al., Proceedings of the National Academy of Sciences U.S.A., 81:5403-5407 (1984), which is specifically incorporated herein by reference.

In construction of the cloning vector of the present invention it should additionally be noted that multiple copies of the synthetic DNA sequence and its attendant operational elements may be inserted into each vector. In such an embodiment the host organism would produce greater amounts per vector of the desired protease inhibitor. The number of multiple copies of the DNA sequence which may be inserted into the vector is limited only by the ability of the resultant vector, due to its size, to be transferred into and replicated and transcribed in an appropriate host microorganism.

Additionally, it is preferred that the vector contain a selectable marker, such as a drug resistance marker or other marker which causes expression of a selectable trait by the host microorganism. In a particularly preferred embodiment of the present invention, the gene for tetracycline resistance is preferably included on the cloning vector.

Such a drug resistance or other selectable marker is intended in part to facilitate in the selection of transformants. Additionally, the presence of such a selectable marker on the cloning vector may be of use in keeping contaminating microorganisms from multiplying in the culture medium. In this embodiment, such a pure culture of the transformed host microorganisms would be obtained by culturing the microorganisms under conditions which require the induced phenotype for survival.

The vector thus obtained is then transferred into the appropriate host microorganism. It is believed that any micororganism having the ability to take up exogenous DNA and express those genes and attendant operational elements may be chosen. It is preferred that the host microorganism be a facultative anaerobe or an aerobe. Particular hosts which may be preferable for use in this method include yeasts and bacteria. Specific yeasts include those of the genus Saccharomyces, and especially Saccharomyces cerevisiae. Specific bacteria include those of the genera Bacillus, Escherichia, and Pseudomonas, especially Bacillus subtilis and Escherichia coli.

After a host organism has been chosen, the vector is transferred into the host organism using methods generally known by those of ordinary skill in the art. Examples of such methods may be found in Advanced Bacterial Genetics by R. W. Davis et al., Cold Spring Harbor Press, Cold Spring Harbor, New York, (1980), which is specifically incorporated herein by reference. It is preferred, in one embodiment, that the transformation occur at low temperatures, as temperature regulation is contemplated as a means of regulating gene expression through the use of operational elements as set forth above. In another embodiment, if osmolar regulators have been inserted into the vector, regulation of the salt concentrations during the transformation would be required to insure appropriate control of the synthetic genes.

If it is contemplated that the recombinant serine protease inhibitors will ultimately be expressed in yeast, it is preferred that the cloning vector first be transferred into Escherichia coli, where the vector would be allowed to replicate and from which the vector would be obtained and purified after amplification. The vector would then be transferred into the yeast for ultimate expression of the serine protease inhibitor.

The host microorganisms are cultured under conditions appropriate for the expression of the serine protease inhibitor. These conditions are generally specific for the host organism, and are readily determined by one of ordinary skill in the art, in light of the published literature regarding the growth conditions for such organisms, for example Bergey's Manual of Determinative Bacteriology, 8th Ed., Williams & Wilkins Company, Baltimore, Maryland, which is specifically incorporated herein by reference.

Any conditions necessary for the regulation of the expression of the DNA sequence, dependent upon any operational elements inserted into or present in the vector, would be in effect at the transformation and culturing stages. In one embodiment, the cells are grown to a high density in the presence of appropriate regulatory conditions which inhibit the expression of the DNA sequence. When optimal cell density is approached, the environmental conditions are altered to those appropriate for expression of the synthetic DNA sequence. It is thus contemplated that the production of the protease inhibitor will occur in a time span subsequent to the growth of the host cells to near optimal density, and that the resultant protease inhibitor will be harvested at some time after the regulatory conditions necessary for its expression were induced.

In a preferred embodiment of the present invention, the recombinant protease inhibitor is purified subsequent to harvesting and prior to assumption of its active structure. This embodiment is preferred as the inventors believe that recovery of a high yield of re-folded protein is facilitated if the protein is first purified. However, in one preferred, alternate embodiment, the protease inhibitor may be allowed re-fold to assume its active structure prior to purification. In yet another preferred, alternate embodiment, the protease inhibitor is present in its re-folded, active state upon recovery from the culturing medium.

In certain circumstances, the protease inhibitor will assume its proper, active structure upon expression in the host microorganism and transport of the protein through the cell wall or membrane or into the periplasmic space. This will generally occur if DNA coding for an appropriate leader sequence has been linked to the DNA coding for the recombinant protein. If the protease inhibitor does not assume its proper, active structure, any disulfide bonds which have formed and/or any noncovalent interactions which have occurred will first be disrupted by denaturing and reducing agents, for example, guanidinium chloride and -mercaptoethanol, before the protease inhibitor is allowed to assume its active structure following dilution and oxidation of these agents under controlled conditions.

It is to be understood that application of the teachings of the present invention to a specific problem or environment will be within the capabilities of one having ordinary skill in the art in light of the teachings contained herein. Examples of the products of the present invention and representative processes for their isolation and manufacture appear in the following example.

### EXAMPLE 1

On the basis of the amino acid sequence described above, the codon usage in highly expressed genes of Escherichia coli, and the provision of convenient restriction endonuclease cleavage sites, the following DNA sequence was proposed:

HindIII
5'AGC GGT AAA AGC TTC AAA GCT GGC GTA TGC CCG CCG
AluI

FnuDII RsaI HpaII
AAA AAA TCC GCG CAG TGT CTG CGG TAC AAA AAA CCG
HhaI

XmaI
GAA TGC CAG TCC GAC TGG CAG TGC CCG GGT AAA AAA
HpaII

NciI

NciI
CGT TGT TGC CCG GAC ACC TGC GGC ATC AAA TGC CTG
HpaII Fnu4HI BstNI

BamHI
GAT CCG GTT GAT ACC CCG AAC CCG ACT CGT CGA AAA
HpaII TaqI

NciI HpaII BalI
CCG GGT AAA TGC CCG GTA ACC TAT GGC CAG TGT CTG
HpaII NciI HaeIII

ATG CTG AAC CCG CCG AAC TTC TGC GAA ATG GAC GGC
HaeIII

BglII
CAG TGT AAA CGA GAT CTG AAA TGC TGT ATG GGT ATG
MboI

Fnu4HI NciI
TGC GGC AAA TCT TGT GTT TCC CCG GTA AAA GCA TAA 3'
HpaII

To regulate the expression of the protein in a form suitable for export to the periplasm of E. coli, the following regulatory elements were proposed: a tac promoter for initiation of transcription at high levels; a lac operator for transcription regulation; a lac repressor (lac I^{q}), to be coded elsewhere on the plasmid; an OmpA Shine-Dalgarno sequence to initiate translation at a high level; an OmpA leader to facilitate periplasmic export of the product; an Ala of an Ala-Ser junction between the protein sequence encoded by these operator elements and that encoded by the structural genes described above to dictate cleavage of the initial product to yield the mature leukocyte elastase inhibitor. All of these features are incorporated into the following DNA sequence:

CTGCA GCTGT TGACA ATTAA TCATC GGCTC GTCTC GTATA ATGTG ATAAC GAGGC
GCAAA AAATG AAAAA GACAG CTATC GCGAT CGCAG TGGCA
CTGGC TGGTT TCGCT ACCGT AGCGC AGGCC.

To regulate the expression of the protein in a form such that the protein remains in the E. coli cytoplasm, the following operational elements are proposed: the tac promoter; the lac operator, and the lac repressor (lac I^{q}); a consensus of Shine-Dalgarno sequences; and, to initiate a high level of translation, a fragment of the OmpA leader peptide to be used as a translational coupler. The translational coupling sequence comprises the DNA coding for the translation initiation region of the OmpA gene, the first eight amino acids of the OmpA leader peptide, the consensus Shine-Dalgarno sequence described above and a translational terminator. The translational coupling sequence is to be inserted between the promoter and the translation initiation site of the serine protease inhibitor gene, overlapping the latter. All of these features are incorporated into the following DNA sequence:

CTGCA GCTGT TGACA ATTAA TCATC GGCTC GTCTC GTATA ATGTG ATAAC GAGGC
GCAAA AATG AAAAA GACAG CTATC GCGAT CGGAG TGTAA GAAAT G.

### A. Construction of Gene Fragments

To construct the above sequences, the following deoxyribonucleotides are synthesized using the ABI DNA synthesizer (Foster City, California). The products are purified by polyacrylamide gel electrophoresis as described in the ABI instrument manual. They are 5' phosphorylated using T4 polynucleotide kinase and ATP using standard means.

The following group of oligonucleotide sequences are used to construct fragment Aa. 
Oligonucleotide Aa1 is:
GCTGT TGACA ATTAA TCAT.

Oligonucleotide Aa2 is:
CGGCT CGTAT AATGT GTGGA ATTGT GAGCG GATAA CAATT T.
Oligonucleotide Aa3 is:
CACAC ATAAC GAGGC GCAAA AA.
Oligonucleotide Aa4 is:
ATGAA AAAGA CAGCT ATCGC GATCG.
Oligonucleotide Aa5 is:
CAGTG GCACT GGCTG GTTTC GCTAC CGTAG CGCAG GCCAG CGGTA AA.
Oligonucleotide Aa6 is:
GAGCC GATGA TTAAT TGTCA ACAGC TGCA.
Oligonucleotide Aa7 is:
TCCGC TCACA ATTCC ACACA TTATA C.
Oligonucleotide Aa8 is:
CCTCG TTATG TGTGA AATTG TTA.
Oligonucleotide Aa9 is:
GCCAC TGCGA TCGCG ATAGC TGTCT TTTTC ATTTT TTGCG.
Oligonucleotide Aa10 is:
AGCTT TTACC GCTGG CCTGC GCTAC GGTAG CGAAA CCAGC CAGT.
 The following oligonucleotide sequences are assembled to make up fragment Ab.

Nucleotide Ab1 is:
GCTGT TGACA ATTAA TCAT.
Nucleotide Ab2 is:
CGGCT CGTAT AATGT GTGGA ATTGT GAGCG GATAA CAATT T.
Nucleotide Ab3 is:
CACAC ATAAC GAGGC GCAAA AA.
Nucleotide Ab4 is:
ATGAA AAAGA CAGCT ATCGC GATCG.
Nucleotide Ab5 is:
GAGTG TAAGA AATGA GCGGT AAA.
Nucleotide Ab6 is:
GAGCC GATGA TTAAT TGTCA ACAGC TGCA.
Nucleotide Ab7 is:
TCCGC TCACA ATTCC ACACA TTATA C.
Nucleotide Ab8 is:
CCTCG TTATG TGTGA AATTG TTA.
Nucleotide Ab9 is:
AGCTT TTACC GCTCA TTTCT TACAC TCCGA TCGCG ATAGC
TGTCT TTTTC ATTTT TTGCG.

The following are the oligonucleotide sequences assembled to construct fragment B.

Oligonucleotide B1 is:
AGCTT CAAAG CTGGC GTATG CCCGC CGAAA AAATC CGCG.
Oligonucleotide B2 is:
CAGTG TCTGC GGTAC AAAAA ACCGG AATGC CAG.
Oligonucleotide B3 is:
TCCGA CTGGC AGTGC CCGGG TAAAA AACGT TGTTG C.
Oligonucleotide B4 is:
CCGGA CACCT GCGGC ATCAA ATGCC TG.
Oligonucleotide B5 is:
GATCC AGGCA TTTGA TGCCG CAGGT GTCCG GGCAA CAACG TTTTT
TACCC GGGCA.
Oligonucleotide B6 is:
CTGCC AGTCG GACTG GCATT CCGGT TTTTT GTACC G.
Oligonucleotide B7 is:
CAGAC ACTGC GCGGA TTTTT TCGGC GGGCA TACGC CAGCT TTGA.

The following are the oligonucleotide sequences used to construct fragment C.

Oligonucleotide C1 is:
GATCC GGTTG ATACC CCGAA CCCG.
Oligonucleotide C2 is:
ACTCG TCGAA AA.
Oligonucleotide C3 is:
CCGGG TAAAT GCCCG GTAAC CTATG GC.
Oligonucleotide C4 is:
CAGTG TCTGA TGCTG AACCC GCCGA AC.
Oligonucleotide C5 is:
TTCTG CGAAA TGGAC GGCCA GTGTA AACGA GAT.
Oligonucleotide C6 is:
CTAGA TCTCG TTTAC ACTGG CCGTC CATTT CGCAG AAGTT.
Oligonucleotide C7 is:
CGGCG GGTTC AGCAT CAGAC ACTGG CCATA GGTTA CCGGG CA.
Oligonucleotide C8 is:
TTTAC CCGGT TTTCG ACGAG TCGGG TT.

Oligonucleotide C9 is:
CGGGG TATCA ACCG.

The following group of oligonucleotide sequences are assembled to form fragment D.

Oligonucleotide D1 is:
GATCT GAAAT GCTGT ATGGG TATGT GCGGC.
Oligonucleotide D2 is:
AAATC TTGTG TTTCC CCGGT AAAAG CATAA G.
Oligonucleotide D3 is:
TCGAC TTATG CTTTT ACCGG GGAAA CACAA GATTT GCCGC A.
Oligonucleotide D4 is:
CATAC CCATA CAGCA TTTCA.

The following groups of oligonucleotides are mixed and annealed under standard conditions and ligated to each other and to cloning and sequencing vectors M13 mp18 and 19 cut with appropriate restriction endonucleases using T4 DNA ligase under standard conditions. The products are used to transform E. coli JM105 and clones containing the DNA of interest are selected from white plaques in IPTG- Xgal plates, and further screened by hybridization with ³²P labelled oligonucleotides selected from the group used in the annealing step. The insert structure is confirmed by dideoxy sequencing of the cloned DNA using a universal primer.

Group Aa contains oligonucleotides Aa1-Aa10 which are ligated to M13 mp18 and 19 cut with PstI and HindIII. Group Ab contains oligonucleotides Ab1-Ab9, which are ligated to M13 mp18 and 19 cut with PstI and HindIII. Group B, which contains oligonucleotides B1 to B7, is ligated-to M13 mp18 and 19 cut with HindIII and BamHI. Group C, which contains oligonucleotides C1 to C9, is ligated to M13 mp18 and 19 cut with BamHI and XbaI. Group D, which contains oligonucleotides D1 to D4, is ligated to M13 mp18 and 19 cut with BamHI and SalI.

M13 replicative form DNA is recovered from the clone having the desired insert DNA by standard means. The insert DNA corresponding to Group Aa is excised from the M13 DNA by cutting the DNA with appropriate restriction endonucleases and is purified by polyacrylamide gel electrophoresis. Its structure is:

AATTCGAGCTCGGTACCCGGGGATCCTCTAGAGTCGACCTGCAGCTG
     GCTCGAGCCATGGGCCCCTAGGAGATCTCAGCTGGACGTCGAC

TTGACAATTAATCATCGGCTCGTATAATGTGTGGAATTGTGAGCG
AACTGTTAATTAGTAGCCGAGCATATTACACACCTTAACACTCGC

GATAACAATTTCACACATAACGAGGCGCAAAAA
CTATTGTTAAAGTGTGTATTGCTCCGCGTTTTT

ATGAAAAAGACAGCTATCGCGATCGCAGTGGCACTGGCT
TACTTTTTCTGTCGATAGCGCTAGCGTCACCGTGACCGA

GGTTTCGCTACCGTAGCGCAGGCCAGC
CCAAAGCGATGGCATCGCGTCCGGTCG

GGTAAA
CCATTTTCGA

The insert DNA corresponding to Group Ab is excised by cutting the DNA with restriction endonucleases EcoRI and HindIII and is purified by polyacrylamide gel electrophoresis. Its structure is:

AATTCGAGCTCGGTACCCGGGGATCCTCTA
GCTCGAGCCATGGGCCCCTAGGAGAT

GAGTCGACCTGCAGCTGTTGACAATTAATC
CTCAGCTGGACGTCGACAACTGTTAATTAG

ATCGGCTCGTATAATGTGTGGAATTGTGAG
TAGCCGAGCATATTACACACCTTAACACTC

CGGATAACAATTTCACACATAACGAGGCGC
GCCTATTGTTAAAGTGTGTATTGCTCCGCG

AAAAAATGAAAAAGACAGCTATCGCGATCGG
TTTTTTACTTTTTCTGTCGATAGCGCTAGCC

AGTGTAAGAAATGAGCGGTAAA
TCACATTCTTTACTCGCCATTTTCGA

The insert DNA corresponding to Group B is excised by cutting the DNA with restriction endonucleases HindIII and BamHI and is purified by polyacrylamide gel electrophoresis. Its structure is:

AGCTTCAAAGCTGGCGTATGCCCGCCG
     AGTTTCGACCGCATACGGGCGGC

AAAAAATCCGCGCAGTGTCTGCGGTACAAA
TTTTTTAGGCGCGTCACAGACGCCATGTTT

AAACCGGAATGCCAGTCCGACTGGCAGTGC
TTTGGCCTTACGGTCAGGCTGACCGTCACG

CCGGGTAAAAAACGTTGTTGCCCGGACACC
GGCCCATTTTTTGCAACAACGGGCCTGTGG

TGCGGCATCAAATGCCTG
ACGCCGTAGTTTACGGACCTAG

The insert DNA corresponding to Group C is excised by cutting the DNA with restriction endonucleoases BamHI and BglII and is purified by polyacrylamide gel electrophoresis. Its structure is:

GATCCGGTTGATACCCCGAACCCGACT
     GCCAACTATGGGGCTTGGGCTGA

CGTCGAAAACCGGGTAAATGCCCGGTA
GCAGCTTTTGGCCCATTTACGGGCCAT

ACCTATGGCCAGTGTCTGATGCTGAACCCG
TGGATACCGGTCACAGACTACGACTTGGGC

CCGAACTTCTGCGAAATGGACGGCCAGTGT
GGCTTGAAGACGCTTTACCTGCCGGTCACA

AAACGA
TTTGCTCTAG

The insert DNA corresponding to Group D is excised by cutting the DNA with restriction endonucleases SauIIIA and SalI and is purified by acrylamide gel electrophoresis. Its structure is:

GATCTGAAATGCTGTATGGGTATG
    ACTTTACGACATACCCATAC

TGCGGCAAATCTTGTGTTTCCCCG
ACGCCGTTTAGAACACAAAGGGGC

GTAAAAGCATAAG
CATTTTCGTATTCAGCT

### B. Construction of the Gene

In the construction for export, the inserts from group Aa, B, C, and D are combined and ligated to M13 mp18 and 19 cut with EcoRI and SalI using T4 DNA ligases under standard conditions. The clones containing the gene are selected by their color on Xgal plates and screened further by hybridization with the ³²P labelled oligonucleotide. The structure of selected clones is confirmed by dideoxy sequencing of the insert region of the DNA using the universal primer.

In the construction for cytoplasmic expression, the inserts from groups Ab, B, C, and D are combined and ligated to M13 mp18 and 19 cut with EcoRI and SalI using T4 DNA ligase under standard conditions. The clones containing the genes are selected by their color on Xgal plates and screened further by hybridization with the ³²P labelled insert. The structures of selected clones are confirmed by dideoxy sequencing of the insert region of the DNA using the universal primer.

### EXAMPLE 2

On the basis of the amino acid sequence described above, the codon usage in highly expressed genes of Escherichia coli, and the provision of convenient restriction endonuclease cleavage sites, the following DNA sequence was proposed:

HindIII
5'TCT GGT AAA AGC TTC AAA GCT GGC GTA TGC CCG CCG
AluI

FnuDII RsaI HpaII
AAA AAA TCC GCG CAG TGT CTG CGG TAC AAA AAA CCG
HhaI

XmaI
GAA TGC CAG TCC GAC TGG CAG TGC CCG GGT AAA AAA
HpaII

NciI

HpaII
CGT TGT TGC CCG GAC ACC TGC GGC ATC AAA TGC CTG
NciI Fnu4HI BstNI

BamHI
GAT CCG GTT GAT ACC CCG AAC CCG ACT CGT CGA AAA
HpaII TaqI

HpaII HpaII BalI
CCG GGT AAA TGC CCG GTA ACC TAT GGC CAG TGT CTG
NciI NciI HaeIII

ATG CTG AAC CCG CCG AAC TTC TGC GAA ATG GAC GGC
HaeIII

BglII
CAG TGT AAA CGA GAT CTG AAA TGC TGT ATG GGT ATG
MboI

Fnu4HI NciI
TGC GGC AAA TCT TGT GTT TCC CCG GTA AAA GCA TAA 3'
HpaII

To regulate the expression of the protein in a form suitable for export to the periplasm of E. coli, the following regulatory elements are proposed: a tac promoter on plasmid pKK223-3 for initiation of transcription at high levels; a lac operator on plasmid pKK223-3 for transcription regulation; a lac repressor (lac I^{q}), to be encoded on the chromosome of E. coli strain JM107; an OmpA Shine-Dalgarno sequence to initiate translation at a high level; an OmpA leader to facilitate periplasmic export of the product; an Ala of an Ala-Ser junction between the protein sequence encoded by these operator elements and that encoded by the structural genes described above to dictate cleavage of the initial product to yield the mature leukocyte elastase inhibitor. The ompA elements are incorporated into the following DNA sequence:

GAATT CGATA TCTCG TTGGA GATAT TCAT GACGT ATTTT GGATG ATAAC GAGGC
GCAAA AAATG AAAAA GACAG CTATC GCGAT CGCAG TGGCA
CTGGC TGGTT TCGCT ACCGT AGCGC AGGCC.

To regulate the expression of the protein in a form such that the protein remains in the E. coli cytoplasm, the following operational elements are proposed: the tac promoter on plasmid pKK223-3; the lac operator of plasmid pKK223-3 and the lac repressor (lac I^{q}) on the chromosome of E. coli strain JM107; a consensus Shine-Dalgarno sequence; and, to initiate a high level of translation, a fragment of the OmpA leader peptide to be used as a translational coupler. The translational coupling sequence comprises the DNA coding for the translation initiation region of the OmpA gene, the first eight amino acids of the OmpA leader peptide, the consensus Shine-Dalgarno sequence described above and a translational terminator. The translational coupling sequence is to be inserted between the lac operator and the translation initiation site of the serine protease inhibitor gene, overlapping the latter. The features of the translational coupler are incorporated into the following DNA sequence:

GAATT CGATA TCTCG TTGGA GATAT TTCAT GACGT ATTTT GGATG ATAAC GAGGC
GCAAA AAATG AAAAA GACAG CTATC GCGAT CAAGG AGAAA TAAAT G.

### C. Construction of Gene Fragments

To construct the above sequences, the following deoxyribonucleotides are synthesized using the ABI DNA synthesizer (Foster City, California). The products are purified by polyacrylamide gel electrophoresis as described in the ABI instrument manual. They are 5' phosphorylated using T4 polynucleotide kinase and ATP using standard means.

The following group of oligonucleotide sequences are used to construct fragment Aa.

Oligonucleotide Aa1 is:
AATTCGATATCTCGTTGGAGATATTCATGACGTATTTTGGATGATAACGAGGCGCAAAA.
Oligonucleotide Aa2 is:
ATGAAAAAGACAGCTATCGCGATCG.
Oligonucleotide Aa3 is:
GATCCGATCGCGATAGCTGTCTTTTTCATTTTTGC.
Oligonucleotide Aa4 is:
GCCTCGTTATCATCCAAAATACGTCATGAATATCTCCAACGAGATATCG.
Oligonucleotide Aa5 is:
GATCCGATCGCAGTGGCACTGGCTGGTTTCGCTACCGTAGCGCAGGCCTCTGGTAAA.
Oligonucleotide Aa6 is:
AGCTTTTACCAGAGGCCTGCGCTACGGTAGCGAAACCAGCCAGTGCCACTGCGATCG.

The following oligonucleotide sequences are assembled to make up fragment Ab.

Oligonucleotide Ab1 is:
AATTCGATATCTCGTTGGAGATATTCATGACGTATTTTGGATGATAACGAGGCGCAAAA.
Oligonucleotide Ab2 is:
ATGAAAAAGACAGCTATCGCGATCG.
Oligonucleotide Ab3 is:
GATCCGATCGCGATAGCTGTCTTTTTCATTTTTGC.
Oligonucleotide Ab4 is:
GCCTCGTTATCATCCAAAATACGTCATGAATATCTCCAACGAGATATCG.
Oligonucleotide Ab5 is:
CAAGGAGAAATAAATGAGCGGTAAA.
Oligonucleotide Ab6 is:

AGCTTTTACCGCTCATTTATTTCTCCTTGAT.

The following are the oligonucleotide sequences assembled to construct fragment B.

Oligonucleotide B1 is:
AGCTT CAAAG CTGGC GTATG CCCGC CGAAA AAATC CGCG.
Oligonucleotide B2 is:
CAGTG TCTGC GGTAC AAAAA ACCGG AATGC CAG.
Oligonucleotide B3 is:
TCCGA CTGGC AGTGC CCGGG TAAAA AACGT TGTTG C.
Oligonucleotide B4 is:
CCGGA CACCT GCGGC ATCAA ATGCC TG.
Oligonucleotide B5 is:
GATCC AGGCA TTTGA TGCCG CAGGT GTCCG GGCAA CAACG TTTTT
TACCC GGGCA.
Oligonucleotide B6 is:
CTGCC AGTCG GACTG GCATT CCGGT TTTTT GTACC G.
Oligonucleotide B7 is:
CAGAC ACTGC GCGGA TTTTT TCGGC GGGCA TACGC CAGCT TTGA.

The following are the oligonucleotide sequences used to construct fragment C.

Oligonucleotide C1 is:
GATCC GGTTG ATACC CCGAA CCCG.
Oligonucleotide C2 is:
ACTCG TCGAA AA.
Oligonucleotide C3 is:
CCGGG TAAAT GCCCG GTAAC CTATG GC.
Oligonucleotide C4 is:
CAGTG TCTGA TGCTG AACCC GCCGA AC.
Oligonucleotide C5 is:
TTCTG CGAAA TGGAC GGCCA GTGTA AACGA GAT.
Oligonucleotide C6 is:
CTAGA TCTCG TTTAC ACTGG CCGTC CATTT CGCAG AAGTT.
Oligonucleotide C7 is:
CGGCG GGTTC AGCAT CAGAC ACTGG CCATA GGTTA CCGGG CA.
Oligonucleotide C8 is:
TTTAC CCGGT TTTCG ACGAG TCGGG TT.
Oligonucleotide C9 is:

CGGGG TATCA ACCG.

The following group of oligonucleotide sequences are assembled to form fragment D.

Oligonucleotide D1 is:
GATCT GAAAT GCTGT ATGGG TATGT GCGGC.
Oligonucleotide D2 is:
AAATC TTGTG TTTCC CCGGT AAAAG CATAA G.
Oligonucleotide D3 is:
TCGAC TTATG CTTTT ACCGG GGAAA CACAA GATTT GCCGC A.
Oligonucleotide D4 is:
CATAC CCATA CAGCA TTTCA.

The following groups of oligonucleotides are mixed and annealed under standard conditions and ligated to each other and to cloning and sequencing vectors M13 mp18 and 19 cut with appropriate restriction endonucleases using T4 DNA ligase under standard conditions. The products are used to transform E. coli JM105 and clones containing the DNA of interest are selected by hybridization with ³²P labelled oligonucleotides selected from the group used in the annealing step. The insert structure is confirmed by dideoxy sequencing of the cloned DNA using a universal primer.

Oligonucleotides Aa1-Aa4 are ligated to M13mp18 and M13mp19 cut with EcoRI and BamHI. M13 replicative form DNA having the desired insert DNA is recovered by standard means. The insert DNA is excised from the M13 DNA by cutting the M13 DNA with restriction endonucleases EcoRI and PvuI and is purified by polyacrylamide gel electrophoresis. Its structure is:

AATTCGATATCTCGTTGGAGATATTCATGACGTATTTTGGATGATAACGAGGCGCAAAAAATGA
    GCTATAGAGCAACCTCTATAAGTACTGCATAAAACCTACTATTGCTCCGCGTTTTTTACT
AAAAGACAGCTATCGCGAT
TTTTCTGTCGATAGCGC

Oligonucleotides Aa5 and Aa6 are ligated to M13mp18 and m13mp19 cut with BamHI and HindIII. M13 replicative form DNA having the desired insert DNA is recovered by standard means. The insert DNA is excised from the M13 DNA by cutting the DNA with restriction endonucleases PvuI and HindIII and is purified by polyacrylamide gel electrophoresis. Its structure is: 
CGCAGTGGCACTGGCTGGTTTCGCTACCGTAGCGCAGGCCTCTGGTAAA
TAGCGTCACCGTGACCGACCAAAGCGATGGCATCGCGTCCGGAGACCATTTTCGA
 This PvuI-HindIII fragment is combined with the EcoRI-PvuI fragment prepared from oligonucleotides Aa1-Aa4 and ligated with M13mp18 or M13mp19 cut with EcoRI and HindIII. M13 replicative form DNA having the desired insert DNA is recovered by standard means. The insert DNA, which is DNA Fragment Aa, is excised from the M13 DNA by cutting the M13 DNA with restriction endonucleases EcoRI and HindIII and is purified by polyacrylamide gel electrophoresis. Its structure is:

AATTCGATATCTCGTTGGAGATATTCATGACGTATTTTGGATGATAACGAGGCGCAAAAAATGA
GCTATAGAGCAACCTCTATAAGTACTGCATAAAACCTACTATTGCTCCGCGTTTTTTACT
AAAAGACAGCTATCGCGATCGCAGTGGCACTGGCTGGTTTCGCTACCGTAGCGCAGGCCTCTGGTA
TTTTCTGTCGATAGCGCTAGCGTCACCGTGACCGACCAAAGCGATGGCATCGCGTCCGGAGACCAT
AA
TTTCGA

Oligonucleotides Ab1-Ab4 are ligated to M13mp18 and M13mp19 cut with EcoRI and BamHI. M13 replicative form DNA having the desired insert DNA is recovered by standard means. The insert DNA is excised from the M13 DNA by cutting the DNA with restriction endonucleases EcoRI and PvuI and is purified by polyacrylamide gel electrophoresis. Its structure is:

AATTCGATATCTCGTTGGAGATATTCATGACGTATTTTGGATGATAACGAGGCGCAAAAAATGA
GCTATAGAGCAACCTCTATAAGTACTGCATAAAACCTACTATTGCTCCGCGTTTTTTACT
AAAAGACAGCTATCGCGAT
TTTTCTGTCGATAGCGC

This EcoRI-PvuI fragment is combined with oligonucleotides Ab5 and Ab6 and ligated with M13mp18 or M13mp19 cut with EcoRI and HindIII. M13 replicative form DNA having the desired insert DNA is recovered by standard means. The insert DNA which is Fragment Ab is excised from the M13 DNA by cutting the DNA with restriction endonucleases EcoRI and HindIII and is purified by polyacrylamide gel electophoresis. Its structure is:

AATTCGATATCTCGTTGGAGATATTCATGACGTATTTTGGATGATAACGAGGCGCAAAAAATGA
GCTATAGAGCAACCTCTATAAGTACTGCATAAAACCTACTATTGCTCCGCGTTTTTTACT
AAAAGACAGCTATCGCGATCAAGGAGAAATAAATGAGCGGTAAA
TTTTCTGTCGATAGCGCTAGTTCCTCTTTATTTACTCGCCATTTTCGA

Group B, which contains oligonucleotides B1 to B7, is ligated to M13mp 18 and 19 cut with HindIII and BamHI. The insert DNA corresponding to Group B is excised by cutting the DNA with restriction endonucleases HindIII and BamHI and is purified by polyacrylamide gel electrophoresis. Its structure is:

AGCTTCAAAGCTGGCGTATGCCCGCCG
AGTTTCGACCGCATACGGGCGGC
AAAAAATCCGCGCAGTGTCTGCGGTACAAA
TTTTTTAGGCGCGTCACAGACGCCATGTTT
AAACCGGAATGCCAGTCCGACTGGCAGTGC
TTTGGCCTTACGGTCAGGCTGACCGTCACG
CCGGGTAAAAAACGTTGTTGCCCGGACACC
GGCCCATTTTTTGCAACAACGGGCCTGTGG
TGCGGCATCAAATGCCTG
ACGCCGTAGTTTACGGACCTAG

Group C, which contains oligonucleotides C1 to C9, is ligated to M13mp 18 and 19 cut with BamHI and XbaI. The insert DNA corresponding to Group C is excised by cutting the DNA with restriction endonucleoases BamHI and BgIII and is purified by polyacrylamide gel electrophoresis. Its structure is:

GATCCGGTTGATACCCCGAACCCGACT
GCCAACTATGGGGCTTGGGCTGA
CGTCGAAAACCGGGTAAATGCCCGGTA
GCAGCTTTTGGCCCATTTACGGGCCAT
ACCTATGGCCAGTGTCTGATGCTGAACCCG
TGGATACCGGTCACAGACTACGACTTGGGC
CCGAACTTCTGCGAAATGGACGGCCAGTGT
GGCTTGAAGACGCTTTACCTGCCGGTCACA
AAACGA
TTTGCTCTAG

Group D, which contains oligonucleotides D1 to D4, is ligated to M13mp 18 and 19 cut with BamHI and SalI. The insert DNA corresponding to Group D is excised by cutting the DNA with restriction endonucleases SauIIIA and SalI and is purified by acrylamide gel electrophoresis. Its structure is:

GATCTGAAATGCTGTATGGGTATG
ACTTTACGACATACCCATAC
TGCGGCAAATCTTGTGTTTCCCCG
ACGCCGTTTAGAACACAAAGGGGC
GTAAAAGCATAAG
CATTTTCGTATTCAGCT

### D. Construction of the Gene

In the construction for export, the inserts from Groups Aa, B, C, and D are combined and ligated to M13 mp18 and 19 cut with EcoRI and SalI using T4 DNA ligase under standard conditions. In the construction for cytoplasmic expression, the inserts from Groups Ab, B, C and D are combined and ligated to M13 mp18 and 19 cut with EcoRI and SalI using T4 DNA ligase under standard conditions. The clones containing the gene are selected by their color on Xgal plates and screened further by hybridization with the ³²P labelled oligonucleotide. The structure of selected clones is confirmed by dideoxy sequencing of the insert region of the DNA using the universal primer.

### EXAMPLE 3

### Construction of Expression Vectors

The inserts for the construction for export and the construction for cytoplasmic expression were transferred to expression plasmids as follows. M13 replicative form DNA having the desired insert DNA is recovered by standard means as indicated above. The appropriate insert DNA is excised from the M13 DNA by cutting the DNA with restriction endonucleases EcoRI and PstI and is purified by polyacrylamide gel electrophoresis. It is then ligated to pKK223-3 cut with restriction endonucleases EcoRI and PstI and the resulting plasmid cloned into E. coli JM107. The construction for use in Examples 4 and 5 for export is pSGE6 and that for use in Example 7 for cytoplasmic expression is pSGE8. The E. coli strain for export in Examples 4 and 5 is SGE10 and that for cytoplasmic expression in Example 6 is SGE30.

### A. Organization of pSGE6

Plasmid pSGE6 was constructed by replacing the DNA between the EcoRI and Pstl sites of pKK223-3 with an EcoRI/PstI fragment containing DNA coding for ompA SLPI. The DNA sequence of ompA-SLPI is as follows:

10 20 30 40 50 60
GAATTCGATA TCTCGTTGGA GATATTCATG ACGTATTTTG GATGATAACG AGGCGCAAAA
CTTAAGCTAT AGAGCAACCT CTATAAGTAC TGCATAAAAC CTACTATTGC TCCGCGTTTT

70 80 90 100 110 120
AATGAAAAAG ACAGCTATCG CGATCGCAGT GGCACTGGCT GGTTTCGCTA CCGTAGCGCA
TTACTTTTTC TGTCGATAGC GCTAGCGTCA CCGTGACCBA CCAAAGCGAT GGCATCGCGT

130 140 150 160 170 180
GGCCTCTGGT AAAAGCTTCA AAGCTGGCGT ATGCCCGCCG AAAAAATCCG CGCAGTGTCT
CCGGAGACCA TTTTCGAAGT TTCGACCGCA TACGGGCGGC TTTTTTAGGC GCGTCACAGA

190 200 210 220 230 240
GCGGTACAAA AAACCGGAAT GCCAGTCCGA CTGGCAGTGC CCGGGTAAAA AACGTTGTTG
CGCCATGTTT TTTGGCCTTA CGGTCAGGCT GACCGTCACG GGCCCATTTT TTGCAACAAC

250 260 270 280 290 300
CCCGGACACC TGCGGCATCA AATGCCTGGA TCCGGTTGAT ACCCCGAACC CGACTCGTCG
GGGCCTGTGG ACGCCGTAGT TTACGGACCT AGGCCAACTA TGGGGCTTGG GCTGAGCAGC

310 320 330 340 350 360
AAAACCGGGT AAATGCCCGG TAACCTATGG CCAGTGTCTG ATGCTGAACC CGCCGAACTT
TTTTGGCCCA TTTACGGGCC ATTGGATACC GGTCACAGAC TACGACTTGG GCGGCTTGAA

370 380 390 400 410 420
CTGCGAAATG GACGGCCAGT GTAAACGAGA TCTGAAATGC TGTATGGGTA TGTGCGGCAA
GACGCTTTAC CTGCCGGTCA CATTTGCTCT AGACTTTACG ACATACCCAT ACACGCCGTT

430 440 450 460
ATCTTGTGTT TCCCCGGTAA AAGCATAAGT CGACCTGCAG
TAGAACACAA AGGGGCCATT TTCGTATTCA GCTGGACGTC

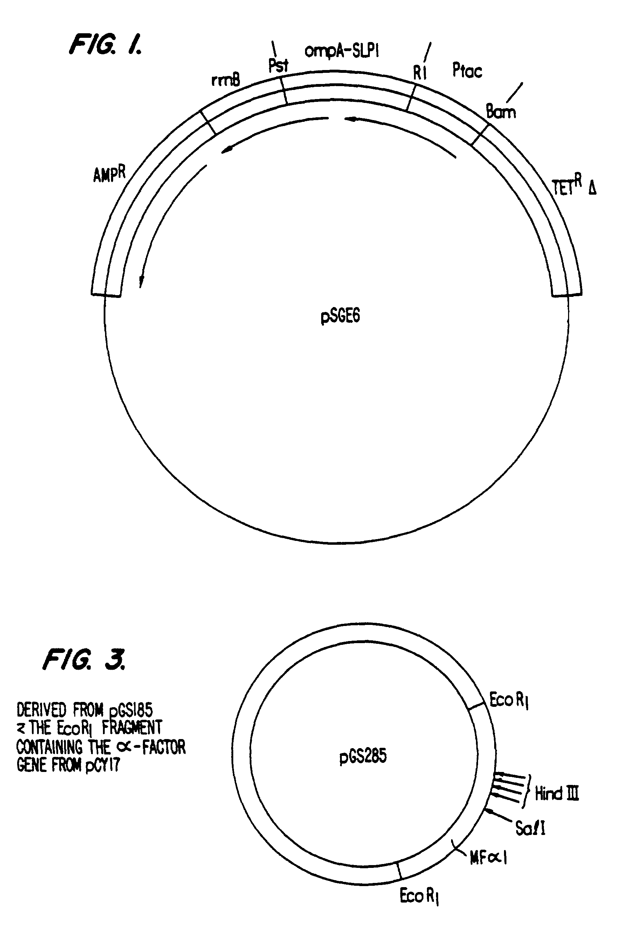

The sequence hereinafter referred to as "ompA-SLPI" is the DNA from the final M13mp18 construct for export discussed above. Plasmid pSGE6 is depicted in Figure 1. In Fig. 1, the first codon for ompAss-SLPI is at position 62-64 of the DNA sequence called "ompA-SLPI." The first codon for mature SLPI is at position 125-127. Ptac contains DNA for the tac promoter, lac operator and the beta galactosidase Shine/Dalgarno sequence. The abbreviations RI, Pst and Bam are recognition sequences for the restriction enzymes EcoRI, PstI and BamHI. Tet^{r} is a part of the gene from pBR322 which confers resistance to tetracycline, amp^{r} confers resistance to ampicillin, rrnB contains the DNA from the rrnB operon from position 6416 to position 6840. Arrows indicate the direction of transcription.

### B. Organization of pCJ-ompA-SLPI

Plasmid pCJ-ompA-SLPI is the same as pSGE6 except that it contains the complete tetracycline resistance gene and promoter rather than the partial gene. This plasmid confers tetracycline resistance when inserted into E. coli and was constructed in a analogous fashion to pSGE6 except that the EcoRI/PstI fragment containing DNA coding for ompA SLPI was cloned into vector pCJI rather than pKK223-3. The vector pCJI was constructed as follows. Plasmid pKK223-3 was digested completely with Sphl and partially with BamHI. A 4.4Kbp fragment was gel purified and combined with a synthetic adaptor: 
GATCTAGAATTGTCATGTTTGACAGCTTATCAT
ATCTTAACAGTACAAACTGTCGAATAGTAGC
 and a 539 bp fragment of DNA from a ClaI, SphI digest of the tet^{r} gene of pBR322 (PL Biochemicals, 27-4891-01).

### C. Structure of pSGE8

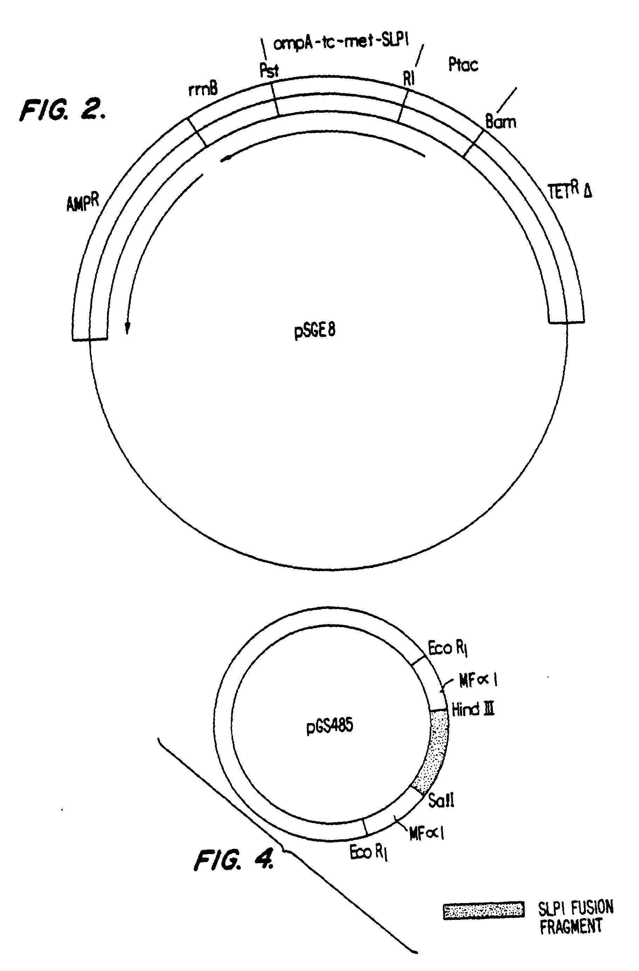

Plasmid pSGE8 is isogenic to pSGE6 with the exception that the DNA between the EcoRI and Pst sites contains the sequence called ompA-tc-met-SLPI which is derived from the final M13mp18 construct for cytoplasmic expression as discussed above. This sequence directs the synthesis of methionyl-SLPI in the cytoplasm of E. coli. A partial diagram of pSGE8 is contained in Fig. 2. In the sequence called "ompA-tc-met-SLPI," the initiation codon for ompA is at position 62-64, the termination codon is at 95-97, and the initiation codon for methionyl-SLPI is at 98-100. The DNA sequence of ompA-tc-met-SLPI is as follows:

10 20 30 40 50 60
GAATTCGATA TCTCGTTGGA GATATTCATG ACGTATTTTG GATGATAACG AGGCGCAAAA
CTTAAGCTAT AGAGCAACCT CTATAAGTAC TGCATAAAAC CTACTATTGC TCCGCGTTTT

70 80 90 100 110 120
AATGAAAAAG ACAGCTATCG CGATCAAGGA GAAATAAATG AGCGGTAAAA GCTTCAAAGC
TTACTTTTTC TGTCGATAGC GCTAGTTCCT CTTTATTTAC TCGCCATTTT CGAAGTTTCG

130 140 150 160 170 180
TGGCGTATGC CCGCCGAAAA AATCCGCGCA GTGTCTGCGG TACAAAAAAC CGGAATGCCA
ACCGCATACG GGCGGCTTTT TTAGGCGCGT CACAGACGCC ATGTTTTTTG GCCTTACGGT

190 200 210 220 230 240
GTCCGACTGG CAGTGCCCGG GTAAAAAACG TTGTTGCCCG GACACCTGCG GCATCAAATG
CAGGCTGACC GTCACGGGCC CATTTTTTGC AACAACGGGC CTGTGGACGC CGTAGTTTAC

250 260 270 280 290 300
CCTGGATCCG GTTGATACCC CGAACCCGAC TCGTCGAAAA CCGGGTAAAT GCCCGGTAAC
GGACCTAGGC CAACTATGGG GCTTGGGCTG AGCAGCTTTT GGCCCATTTA CGGGCCATTG

310 320 330 340 350 360
CTATGGCCAG TGTCTGATGC TGAACCCGCC GAACTTCTGC GAAATGGACG GCCAGTGTAA
GATACCGGTC ACAGACTACG ACTTGGGCGG CTTGAAGACG CTTTACCTGC CGGTCACATT

370 380 390 400 410 420
ACGAGATCTG AAATGCTGTA TGGGTATGTG CGGCAAATCT TGTGTTTCCC CGGTAAAAGC
TGCTCTAGAC TTTACGACAT ACCCATACAC GCCGTTTAGA ACACAAAGGG GCCATTTTCG

430
ATAAGTCGAC CTGCAG
TATTCAGCTG GACGTC

### D. Organization of pCJ-met-SLPI

Plasmid pCJ-met-SLPI is the same as pSGE8 except that it contains the complete (rather than the partial) tetracycline resistance gene. Plasmid CJ-met-SLPI was constructed analogously to pSGE8 except that the EcoRI/PstI fragment containing DNA coding for ompA-tc-met-SLPI was cloned into vector pCJI rather than pKK223-3.

### E. Construction of Yeast Expression Plasmids

The plasmid pUC8 was digested with HindIII and ligated to a HindIII/SmaI adaptor (obtained from Amersham, Cat. No. DA1006). The addition of this adaptor to a HindIII site does not reconstruct the HindIII site. The DNA was then digested with Smal and ligated in dilute solution followed by tranformation of E. coli JM83. The correct plasmid, i.e., a plasmid lacking the restriction sites in the polylinker from the HindIII site to the SmaI site, was identified by digesting plasmid DNA isolated from transformants with EcoRI, Smal or HindIII. A transformant containing a plasmid that lacked the HindIII site but contained the EcoRI site and Smal site was identified in this manner. This plasmid is pGS185.

An EcoRI fragment containing the yeast MF I gene was purified by gel electrophoresis from the plasmid pCY17 as described by J. Kurjan & I. Herskowitz in Cell 30:933 (1982), specifically incorporated herein by reference, and ligated into EcoRI cut pGS185. This ligation mixture was used to transform E. coli HB101, selecting for ampicillin resistance. Plasmid DNA was isolated from transformants and the presence of the correct insert confirmed by digests of the DNA with EcoRI. This is plasmid pGS285 and is depicted in Fig. 3.

Plasmid pGS285 was digested to completion with HindIII and religated under dilute conditions to eliminate three of the four internal HindIII sites in the MF I gene as noted by Kurjan & Herskowitz, ibid. The correct construct was selcted as described above. This is plasmid pGS385.

The M13 AaBCD clone as described in Example 2 that carries nucleotide sequences encoding amino acids four through 107 of the synthetic SLPI gene, was digested with HindIII. This DNA was ligated with the following oligonucleotide adaptor: 
5' GCT GAA GCT TCA GGT AAG
CGA CTT CGA AGT CCA TTC TCGA.
 This adaptor had been formed by annealing the two oligonucleotides:
5' GCT GAA GCT TCA GGT AAG and 5' AGC TCT TAC CTG AAG CTT CAGC first at 70 ° C for 2' followed by slow cooling overnight.

Following ligation of the adaptor to HindIII cut M13 AaBCD, the ligation mix was digested with HindIII and Sail to release a fragment purified by agarose gel electrophoresis and electrolution. This fragment was digested once more with HindIII and then ligated with pGS385 DNA that had been cut with HindIII and SalI. E. coli HB101 was transformed with the ligation mixture and ampicillin resistant transformants were selected. Transformants containing plasmids with the correct insert DNA were identified by preparing plasmid DNA and digesting it with HindIII and SalI. A plasmid constructed and isolated in this manner has been designated pGS485 and is depicted in Figure 4. This plasmid contains the MF I gene fused, in frame, to the synthetic SLPI gene at the HindIII site in the first spacer region of the MF I gene. Such constructs, when placed in yeast, have been demonstrated to direct the synthesis, processing and secretion of the heterologous proteins as shown by A.J. Brake et al. in PNAS (USA) 81:4642, specifically incorporated herein by reference. The fusion of the MF I gene and SLPI is contained on an EcoRI fragment in pGS485. This EcoRI fragment was cloned into the vector YIp5 as described in Example 8.

### EXAMPLE 4

Expression and purification of secretory leukocyte protease inhibitor (SLPI) using plasmid pSGE6. E. coli cells containing plasmid pSGE6 (SGE10 cells) were cultured for 6 hours in 10 liters of M9 media with 2% tryptone, 0.5% yeast extract, 20g/l glucose, 200 mg/l Vitamin B₁ and 100 mg/l ampicillin added. IPTG was added to 0.2mM and the culture grown for another 6 hours. Ten liters of E. coli SGE10 cells, at 8 grams per liter, were pelleted at 18,000 x g and resuspended in 50mM Tris.HCl (pH 7.5), 4mM EDTA buffer (hereinafter T50E4) and pelleted. The pellet was resuspended in 2.7 liters T50E4 and frozen in 150ml lots. Eight of these lots (equivalent to 36 gms of cells) were pooled and lysed by a single pass through a french press at 12,000 psi and 4° C. The lysate was centrifuged for 1.5hrs at 20,000 x g. One sixth of the pellet containing the cell insolubles (equivalent to six grams of cells) was washed twice with 125ml of T50E4 and the remaining material was frozen overnight.

The frozen pellet was extracted with 25ml of 100mM Tris.HCl (pH 8.0), 4mM EDTA (hereinafter T100E4) containing 20mM DTT (obtained from Sigma, Cat. No. D-0632), 4mM PMSF (obtained from Sigma, Cat. No. P-7626) and 8M urea (ultrapure, obtained from BRL, Cat. No. 5505UA) for 1hr at 37° C. and centrifuged at 10,000 x g for ten minutes. The resultant supernatant was mixed with 10ml packed Sephadex SP-C25 (obtained from Pharmacia) which had been pre-equilibrated with the extraction buffer T100E4 containing 20mM DTT and 8M urea and mixed on a roller for ten minutes at 37°C to absorb the SLPI to the SP-Sephadex.

The resin with the absorbed SLPI was pelleted by a ten minute centrifuge at 3,000 x g and the supernatant decanted. The remaining resin was washed twice with 25ml of T100E4 containing 20mM DTT and 8M urea followed by two washes with 25ml T100E4 containing 20mM DTT. The resin was then extracted once with a mixture of 0.6ml 5 M NaCI and 25ml of T100E4 containing 20 mM DTT and 0.3 M NaCI. This extract contained about 0.15mg/ml protein and more than 0.04mg/ml SLPI. The SLPI obtained by this method was determined to be greater than 70% pure by high pressure liquid chromatography.

### EXAMPLE 5

Using the method of Example 4, a second frozen pellet was extracted with T100E4 containing 1% Triton x-100 (obtained from Sigma, Cat. No. T-6878) in place of the first T100E4/DTT/PMSF/urea wash. The resultant SLPI was slightly more pure than that obtained in Example 4 and gave higher activity in the refolding assay set forth in Example 6 below.

### EXAMPLE 6

### Refolding purified SLPI.

About 40ug of partially-purified SLPI from Example 4 or 5 was made 8M in urea or 5M in guanidine hydrochloride (obtained from Pierce Chemical Co., #24110), and 4mM in DTT and incubated for 1hr at room temperature. Oxidized glutathione (obtained from Sigma, Cat. No. G-4626) was added to 13.5mM and the mixture was again incubated for 1 hr at room temperatrue. The mixture was diluted 10-fold with a solution of 50mM Tris in NaOH, pH10.7 and incubated for a further 4 hrs at room temp. The mixture was then diluted 5-fold with 50mM Tris, pH8.0, and 0.15M NaCI and applied to a 1 x 2 cm column of Sepahdex SP-C25 preequilibrated with 50mM Tris, pH8.0 and 0.25M NaCI. The resin was washed with 50mM Tris, pH 8.0, containing 0.25M NaCl and then with 50mM Tris, pH8.0, containing 0.5M NaCI. The fraction eluting with the 0.5M salt wash was fully active and represented about 30% of the SLPI applied to the column.

### EXAMPLE 7

Purification of SLPI from soluble and insoluble fractions of SGE30 cell lysate.

Expression of the plasmid pSGE8 in E. coli SGE30 cells produced SLPI in both the soluble and insoluble fractions of the cell lysate. At 1% of the total cell protein, the SLPI was distributed about 80% to the soluble and about 20% to the insoluble fractions.

### A. Purification of SLPI from the insoluble fraction

The E. coli SGE30 cells containing pSGE8 were grown in LB Media containing 50 ug/ml ampicillin in a shaker flask to an OD600 of 0.7 and induced by the addition of IPTG to 0.2mM. After three hours the cells are pelleted and were suspended in two

### A. Purification of SLPI from the insoluble fraction

The E. coli SGE30 cells containing pSGE8 were grown in LB Media containing 50 ug/ml ampicillin in a shaker flask to an OD600 of 0.7 and induced by the addition of IPTG to 0.2mM. After three hours the cells are pelleted and were suspended in two times their weight of 50mM Tris.HCl (pH 7.5) and 4mM EDTA (hereinafter T50E4). The cells were disrupted by sonication at 4 ° C. and the extract was centrifuged for 20 minutes at 4° C. at 12,000 x g.

The pellet was washed in three volumes of T50E4 and was solublized at room temperature in a solution containing either 10M urea or 6M guanidine hydrochloride, and 5mM reduced DTT. After a one hour incubation at room temperature, oxidized glutathione was added at a concentration of 17.5mM and the mixture was incubated for another hour. The mixture was then diluted into 10 volumes of 50 mM Tris.HCl, pH 10.7. The diluted mixture was allowed to stand for 4 hours at room temperature followed by pH adjustment to 8 by the addition of 5 N HCI. This mixture was centrifuged to remove precipitated protein.

The supernatant so produced contained SLPI which exhibited secretory leukocyte protease inhibitor activity. This protein was purified by chromatography on a Sephadex SP-C25 column as described above.

### B. Purification of SLPI from soluble fraction

E. coli SGE30 cells containing plasmid pSGE8 were grown in a shaker flask to an 00600 of 0.7 and induced by the addition of IPTG to 0.2mM. At an 00600 of 1.1, the cells were pelleted at 25,000 x g for 15 minutes. The pellet was resuspended in T50E4 and was lysed by two passages through a french press at 20,000 psi at 4° C. The lysate was centrifuged at 25,000 x g for 15 minutes.

The supernatant was made 25mM in DTT. This mixture was incubated at 0° C. for one hour and sufficient HCI was added to reach a final concentration of 5%. After a 30 minute incubation at 0° C., the mixture was centrifuged at 25,000 x g for 15 minutes and the supernatant removed for further processing. The pH of the supernatant was adjusted to 8.0 with 10M NaOH and analyzed by sds-page, reverse phase hplc chromatography and elisa which indicated at least 0.7 ug slpi per 130 ug total protein. the slpi thus obtained was further purified on a sephadex sp-c25 chromatography column. it was refolded to active slpi according to example 6.

### EXAMPLE 8

The EcoRI fragment containing the fused SLPI-MF I gene (see Ex. 3.E.) was ligated to the EcoRI site of the yeast vector Ylp5 as described by D. Botstein and R.W. Davis in The Molecular Biology of the Yeast Saccharomyces, Cold Spring Harbor Laboratory, pp. 607-636 (1982), specifically incorporated herein by reference, to generate YlpSLPI-I and has been integrated into the URA3 gene of S. cerevisiae BS214 (MAT , Ura3-52, pep4, prbl) by site-directed recombination as described by T. Orr-Weaver et al. in Methods in Enzymology 101:228 (1983), specifically incorporated herein by reference. This strain, S. cerevisiae SGY-1, secretes fully active SLPI into the culture supernate.

A second strain, SGY-3, also produces and secretes active SLPI. This strain carries the MF I::SLPI fusion on the replicating yeast plasmid pGS585. This plasmid was constructed from pJDB207 as described by J.R. Broach in Methods in Enzymology 101:307 (1983), specifically incorporated herein by reference, by the addition of the yeast URA3 gene, isolated from the plasmid YEp24 as described by D. Botstein and R.W. Davis in The Molecular Biology of the Yeast Saccharomyces, Cold Spring Harbor Laboratory, pp. 607-636, specifically incorporated herein by reference, and cloned into the HindIII site of pJDB207 to construct pGS585. The MF I::SLPI fusion gene, contained on an EcoRI fragment, was cloned into the SalI site of pGS585 using EcoRI-XhoI adaptors (obtained from Amersham, Cat. No. DA1007) to generate YEpSLPI-I. This plasmid was introduced into S. cerevisiae DBY746 (MAT , Ura3-52, leu2-3, his3 1, trp 1-289) by transformation as described by Ito et al. in J. Bacteriology 153:163 (1983), specifically incorporated herein by reference.

Saccharomyces cerevisiae strains SGY-1 and SGY-3 were grown at 30°C to stationary phase in SD medium lacking uracil according to the method of F. Sherman et al. described in Methods in Yeast Genetics, p. 62, Cold Spring Harbor Laboratories, Cold Spring Harbor, New York (1981), specifically incorporated herein by reference. Cells were removed from the culture medium by centrifugation and the culture supernatant was assayed for SLPI activity by measuring (1) protease inhibitory activity and (2) the amount of material that specifically reacts with anti-SLPI antibodies by an enzyme-linked immunoassay. Purification schemes may be developed in a manner analogous to prior methods described herein.

## Claims (Claims for the following Contracting State(s): BE, CH, LI, DE, FR, GB, IT, LU, NL, SE)

1. A method for the production of a recombinant serine protease inhibitor comprising a single unfragmented polypeptide chain and having at least one active site possessing serine protease inhibitor activity, said inhibitor having the following amino acid sequence: 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
 said method comprising the steps of:
a) culturing a host microorganism transformed with a DNA sequence capable of directing production of the serine protease inhibitor under conditions appropriate for expression of the DNA sequence, and;
b) harvesting the inhibitor.

2. The method of claim 1 further comprising the step of permitting the inhibitor to assume an active tertiary structure whereby it possesses serine protease inhibitor activity.

3. The method of claim 1 wherein said DNA sequence is a synthetic DNA sequence.

4. The method of claim 1 wherein said DNA sequence is a natural DNA sequence.

5. The method of claim 1 which further comprises purification of the inhibitor.

6. The method of claim 5 wherein said purification is conducted prior to permitting said inhibitor to assume an active form.

7. The method of claim 5 wherein purification is conducted subsequent to permitting said inhibitor to assume an active form.

8. The method of claim 1 wherein the DNA sequence is carried on a vector comprising portions of the vectors selected from the group consisting of pBR322 and pIQ.

9. The method of claim 1 wherein the host microorganism is selected from the group consisting of microorganisms of the genera Escherichia, Bacillus and Saccharomyces.

10. The method of claim 9 wherein the host microorganism is Escherichia coli.

11. The method of claim 9 wherein the host microorganism is Bacillus subtilis.

12. The method of claim 9 wherein the host microorganism is Saccharomyces cerevisiae.

13. The method of claim 4 wherein the natural DNA sequence is obtained by a method comprising the steps of:
a) Preparing a human cDNA library from cells capable of expressing the native serine protease inhibitor;
b) Probing the cDNA library with at least one nucleic acid probe capable of hybridizing to a cDNA coding for the inhibitor or with at least one probe capable of binding to protease inhibitor protein expressed by a clone of the cDNA library;
c) identifying at least one clone containing a cDNA coding for the inhibitor and, optionally;
d) Isolating the cDNA coding for the inhibitor from the clone; and
e) Linking the cDNA, or fragments thereof, to operational elements to maintain and express the cDNA in a host microorganism.

14. The method of claim 13 wherein the cells are human parotid cells.

15. The method of Claim 4 wherein said natural DNA sequence is obtained by a method comprising the steps of:
a) Preparing a human genomic DNA library;
b) Probing the genomic DNA library with at least one nucleic acid probe capable of hybridizing to a genomic DNA coding for the inhibitor or with at least one probe capable of binding to protease inhibitor protein expressed by a clone of the genomic DNA library;
c) Identifying at least one clone containing a genomic DNA coding for the inhibitor and, optionally;
d) Isolating the genomic DNA coding for the inhibitor from the clone; and
e) Linking the genomic DNA, or fragments thereof, to operational elements to maintain and express the DNA in a host microorganism.

16. A synthetic DNA sequence capable of directing microbial synthesis of a serine protease inhibitor comprising a single unfragmented polypeptide chain having at least one active site possessing serine protease inhibitor activity, said inhibitor having the following amino acid sequence:

Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala.

17. The DNA sequence of claim 16 wherein said sequence is selected from the group consisting of (1) 
5' AGCGG TAAAA GCTTC AAAGC TGGCG TATGC CCGCC GAAAA AATCC GCGCA GTGTC
TGCGG TACAA AAAAC CGGAA TGCCA GTCCG ACTGG CAGTG CCCGG GTAAA AAACG TTGTT
GCCCG GACAC CTGCG GCATC AAATG CCTGG ATCCG GTTGA TACCC CGAAC CCGAC TCGTC
GAAAA CCGGG TAAAT GCCCG GTAAC CTATG GCCAG TGTCT GATGC TGAAC CCGCC GAACT
 
TCTGC GAAAT GGACG GCCAG TGTAA ACGAG ATCTG AAATG CTGTA TGGGT ATGTG CGGCA
AATCT TGTGT TTCCC CGGTA AAAGC ATAA 3';
 or (2) sequences which are equivalent to (1) as a result of degeneracy of the genetic code.

18. A recombinant vector comprising the DNA sequence of Claims 16 or 17.

19. A host cell transformed with the recombinant vector of Claim 18.

20. An isolated DNA sequence which codes for a serine protease inhibitor comprising a single unfragmented polypeptide chain having the following amino acid sequence:

Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala.

21. The DNA sequence of Claim 20 which is a cDNA.

22. The DNA sequence of Claim 20 which is a genomic DNA.

23. A recombinant vector comprising the DNA sequence of Claims 20, 21, or 22.

24. A host cell transformed with the recombinant vector of Claim 23.

25. A method for the synthesis of a recombinant serine-protease inhibitor comprising a single unfragmented polypeptide chain, such a method comprising:
a) Preparing a DNA sequence capable of directing a host microorganism to produce a polypeptide comprising the amino acid sequence: 
R1 -Gly-Lys-Ser-Phe-Lys-Ala-Gly-Val-Cys-Pro-Pro-Lys-Lys-Ser-Ala-Gln-Cys-Leu-
R2 -Tyr-Lys-Lys-Pro-Glu-Cys-Gln-Ser-Asp-Trp-Gln-Cys-Pro-Gly-Lys-Lys-Arg-Cys-
 
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys- R8- R3- R9-Asn-Pro-
Pro-Asn-Phe-Cys-Glu- R4-Asp-Gly-Gln-Cys-Lys-Arg-Asp-Leu-Lys-Cys-Cys- R5-Gly-
R6 -Cys-Gly-Lys-Ser-Cys-Val-Ser-Pro-Val-Lys- R7,
 or a fragment of said DNA encoding a polypeptide having at least one active site possessing serine protease inhibitor activity,
wherein
R1 and R7 are the same or different and are selected from the group consisting of a substituted or unsubstituted amino acid residue or derivative thereof; and
R2, R3, R4, R5, R6, R8 and R9 are the same or different and are selected from the group consisting of methionine, valine, alanine, phenylalanine, tyrosine, tryptophan, lysine, glycine and arginine;
b) Cloning the DNA sequence into a vector capable of being transferred into and replicating in a host microorganisms such vector containing operational elements for the DNA sequence;
c) Transferring the vector containing the DNA sequence and operational elements into a host microorganism capable of expressing the serine protease inhibitor;
d) Culturing the host microorganism under conditions appropriate for amplification of the vector and expression of the inhibitor;
e) Harvesting the inhibitor; and
f) Permitting the inhibitor to assume an active tertiary structure whereby it possesses serine protease inhibitor activity.

26. A method according to claim 25 wherein said single unfragmented polypeptide chain serine protease inhibitor comprises the amino acid sequence: 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
 or a fragment thereof, said fragment having at least one active site possessing serine protease inhibitor activity.

27. A DNA sequence capable of directing microbial synthesis of a single unfragmented polypeptide chain wherein said polypeptide comprises the amino acid sequence: 
R1-Gly-Lys-Ser-Phe-Lys-Ala-Gly-Val-Cys-Pro-Pro-Lys-Lys-Ser-Ala-Gln-Cys-Leu-
R2-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys- R8- R3-R9-Asn-Pro-
Pro-Asn-Phe-Cys-Glu-R4-Asp-Gly-Gln-Cys-Lys-Arg-Asp-Leu-Lys-Cys-Cys- R5-Gly-
R6-Cys-Gly-Lys-Ser-Cys-Val-Ser-Pro-Val-Lys- R7,
 or a fragment of said DNA encoding a polypeptide having at least one active site possessing serine protease inhibitor activity; wherein
R1 and R7 are the same or different and are selected from the group consisting of a substituted or unsubstituted amino acid residue or derivative thereof; and R2, R3, R4, R5, R6, R8 and R9 are the same or different and are selected from the group consisting of methionine, valine, alanine, phenylalanine, tyrosine, tryptophan, lysine, glycine and arginine.

28. A DNA sequence of claim 27, wherein said serine protease inhibitor comprises the amino acid sequence: 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
 or a fragment thereof, said fragment having at least one active site possessing serine protease inhibitor activity.

29. A recombinant vector comprising the DNA sequence of claim 27 or 28.

30. A host cell transformed with the recombinant vector of claim 29.

## Claims (Claims for the following Contracting State(s): AT)

1. A method for the production of a recombinant serine protease inhibitor comprising a single unfragmented polypeptide chain, said inhibitor having at least one active site possessing serine protease inhibitor activity said inhibitor having the following amino acid sequence: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala.
 said method comprising the steps of:
a) culturing a host microorganism transformed with a DNA sequence capable or directing production of the serine protease inhibitor under conditions appropriate for expression of the DNA sequence, and;
b) harvesting the inhibitor.

2. The method of Claim 1 further comprising the step of permitting the inhibitor to assume an active tertiary structure whereby it possesses serine protease inhibitor activity.

3. The method of Claim 1 wherein said DNA sequence is a synthetic DNA sequence.

4. The method of Claim 1 wherein said DNA sequence is a natural DNA sequence.

5. The method of Claim 1 which further comprises purification of the inhibitor.

6. The method of Claim 5 wherein said purification is conducted prior to permitting said inhibitor to assume an active form.

7. The method of Claim 5 wherein purification in conducted subsequent to permitting said inhibitor to assume an active form.

8. The method of Claim 1 wherein the DNA sequence is carried on a vector comprising portions of the vectors selected from the group consisting of pBR322 and pIQ.

9. The method of Claim 1 wherein the host microorganism is selected from the group consisting of microorganisms of the genera Escherichia, Bacillus and Saccharomyces.

10. The method of Claim 9 wherein the host microorganism is Escherichia coli.

11. The method of Claim 9 wherein the host microorganism is Bacillus subtilis.

12. The method of Claim 9 wherein the host microorganism is Saccharomyces cerevisiae.

13. The method of Claim 4 wherein the natural DNA sequence is obtained by a method comprising the steps of:
a) Preparing a human cDNA library from cells capable of expressing the native serine protease inhibitor;
b) Probing the cDNA library with at least one nucleic acid probe capable of hybridizing to a cDNA coding for the inhibitor or with at least one probe capable of binding to protense inhibitor protein expressed by a clone of the cDNA library;
c) Identifying at least one clone containing a cDNA coding for the inhibitor and, optionally;
d) Isolating the cDNA coding for the inhibitor from the clone; and
e) Linking the cDNA, or fragments thereof, to operational elements to maintain and express the cDNA in a host microorganism.

14. The method of Claim 13 wherein the cells are human parotid cells.

15. The method of Claim 4 wherein said natural DNA sequence is obtained by a method comprising the steps of:
a) Preparing a human genomic DNA library;
b) Probing the genomic DNA library with at least one nucleic acid probe capable of hybridizing to a genomic DNA coding for the inhibitor or with at least one probe capable of binding to protease inhibitor protein expressed by a clone of the genomic DNA library;
c) Identifying at least one clone containing a genomic DNA coding for the inhibitor and, optionally;
d) Isolating the genomic DNA coding for the inhibitor from the clone; and
e) Linking the genomic DNA, or fragments thereof, to operational elements to maintain and express the DNA in a host microorganism.

16. A method for the production of a synthetic DNA sequence capable of directing microbial synthesis of a serine protease inhibitor comprising a single unfragmented polypeptide chain, said inhibitor having at least one active site possessing serine protease inhibitor activity said inhibitor having the following amino acid sequence: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 comprising preparing said synthetic DNA sequence by polynucleotide synthesis in a manner known per se.

17. The method of Claim 16 wherein said sequence is selected from the group consisting of (1): 
5'	AGCGG TAAAA GCTTC AAAGC TGGCG TATGC
CCGCC GAAAA AATCC GCGCA GTGTC TGCGG
TACAA AAAAC CGGAA TGCCA GTCCG ACTGG
CAGTG CCCGG GTAAA AAACG TTGTT GCCCG
GACAC CTGCG GCATC AAATG CCTGG ATCCG
GTTGA TACCC CGAAC CCGAC TCGTC GAAAA
CCGGG TAAAT GCCCG GTAAC CTATG GCCAG
TGTCT GATGC TGAAC CCGCC GAACT TCTGC
GAAAT GGACG GCCAG TGTAA ACGAG ATCTG
AAATG CTGTA TGGGT ATGTG CGGCA AATCT
TGTGT TTCCC CGGTA AAAGC ATAA 3'
 or (2) sequences which are equivalent to (1) an a result of degeneracy of the genetic code.

18. A method for the production of a recombinant vector which comprises the DNA sequence obtained by the method of Claim 16 or 17 comprising inserting said DNA sequence into a suitable vector.

19. A method for the production of a host cell transformed with the recombinant vector obtained by the method of Claim 18 comprising transforming a host cell with said vector.

20. A method for the production of a natural DNA sequence which codes for a serine protease inhibitor comprising a single unfragmented polypeptide chain having the following amino acid sequence: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala.
 comprising the steps of
a) Preparing a human cDNA library from cells capable of expressing the native serine protease inhibitor;
b) Probing the cDNA library with at least one nucleic acid probe capable of hybridizing to a cDNA coding for the inhibitor or with at least one probe capable of binding to protease inhibitor protein expressed by a clone of the cDNA library;
c) Identifying at least one clone containing a cDNA coding for the inhibitor and,
d) Isolating the cDNA coding for the inhibitor from the clone;
or
a) Preparing a human genomic DNA library;
b) Probing the genomic DNA library with at least one nucleic acid probe capable of hybridizing to a genomic DNA coding for the inhibitor or with at least one probe capable of binding to protease inhibitor protein expressed by a clone of the genomic DNA library;
c) Identifying at least one clone containing a genomic DNA coding for the inhibitor and,
d) Isolating the genomic DNA coding for the inhibitor from the clone.

21. The method of Claim 20 wherein the DNA sequence is a cDNA.

22. The method of Claim 20 therein the DNA sequence is a genomic DNA.

23. A method for the production of a recombinant vector which comprises the DNA sequence obtained by the method of Claims 20, 21 or 22 comprising inserting said DNA sequences into a suitable vector.

24. A method for the production of a host cell transformed with the recombinant vector obtained by the method of Claim 23 comprising transforming a host cell with said vector.

25. A method for the synthesis of a recombinant serine protease inhibitor comprising a single unfragmented polypeptide chain, such a method comprising:
a) Preparing a DNA sequence capable of directing a host microorganism to produce a potypeptide comprising the amino acid sequence: 
R1-Gly-Lys-Ser-Phe-Lys-Ala-Gly-Val-Cys-Pro-
Pro-Lys-Lys-Ser-Ala-Gln-Cys-Leu-R2-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-R8-R3-R9-Asn-Pro-Pro-
Asn-Phe-Cys-Glu-R4-Asp-Gly-Gln-Cys-Lys-Arg-
Asp-Leu-Lys-Cys-Cys-R5-Gly-R6-Cys-Gly-Lys-
Ser-Cys-Val-Ser-Pro-Val-R7,
 or a fragment of said DNA encoding a polypeptide having at least one active site possessing serine protease inhibitor activity, wherein
R₁ and R₇ are the same or different and are selected from the group consisting of a substituted or unsubstituted amino acid residue or derivative thereof; and
R₂, R₃, R₄, R₅, R₆, R₈ and R₉ are the same or different and are selected from the group consisting of methionine, valine, alanine, phenylalanine, tyrosine, tryptophan, lysine, glycine and arginine,
b) Cloning the DNA Sequence into a vector capable of being transferred into and replicating in a host microorganism, such vector containing operational elements for the DNA sequence;
c) Transferring the vector containing the DNA sequence and operational elements into a host microorganism capable of expressing the serine protease inhibitor;
d) Culturing the host microorganism under conditions appropriate for amplification of the vector and expression of the inhibitor;
e) Harvesting the inhibitor; and
f) Permitting the inhibitor to assume an active tertiary structure whereby it possesses serine protease inhibitor activity.

26. A method according to claim 25 wherein said single unfragmented polypeptide chain serine protease inhibitor comprises the amino acid sequence: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 or a fragment thereof, said fragment having at least one active site possessing serine protease inhibitor activity.

27. A method for the Production of a DNA sequence capable of directing microbial synthesis of a single unfragmented polypeptide chain wherein said polypeptide comprises the amino acid sequence: 
R1-Gly-Lys-Ser-Phe-Lys-Ala-Gly-Val-Cys-Pro-
Pro-Lys-Lys-Ser-Ala-Gln-Cys-Leu-R2-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-R8-R3-R9-Asn-Pro-Pro-
Asn-Phe-Cys-Glu-R4-Asp-Gly-Gln-Cys-Lys-Arg-
Asp-Leu-Lys-Cys-Cys-R5-Gly-R6-Cys-Gly-Lys-
Ser-Cys-Val-Ser-Pro-Val-Lys-R7,
 or a fragment of said DNA encoding a polypeptide having at least one active site possessing serine protease inhibitor activity;
wherein
R₁ and R₇ are the same or different and are selected from the group consisting of a substituted or unsubstituted amino acid residue or derivative thereof; and
R₂, R₃, R₄, R₅, R₆, R₈ and R₉ are the same or different and are selected from the group consisting of methionine, valine, alanine, phenylalanine, tyrosine, tryptophan, lysine, glycine and arginine, comprising preparing said DNA sequence in a manner known per se.

28. The method of Claim 27, wherein said serine protease inhibitor comprises the amino acid sequence: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 or a fragment thereof, said fragment having at least one active site possessing serine protease inhibitor activity.

29. A method for the production of a recombinant vector which comprises the DNA sequence obtained by the method of Claim 27 or 28 comprising inserting said DNA sequence into a suitable vector.

30. A method for the production of a host cell transformed with the recombinant vector obtained by the method of Claim 29 comprising transforming a host cell with said vector.

## Patentansprüche (Patentansprüche für folgende(n) Vertragsstaat(en): BE, CH, LI, DE, FR, GB, IT, LU, NL, SE)

1. Verfahren zur Herstellung eines rekombinanten Serinprotease-Inhibitors, der eine einzelne, unfragmentierte Polypeptidkette umfasst und mindestens eine aktive Stelle aufweist, die Serinprotease-Inhibitoraktivität besitzt, wobei der Inhibitor die folgende Aminosäuresequenz aufweist: 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
 worin das Verfahren die folgenden Schritte umfaßt:
a) Kultivieren eines Wirtsmikroorganismus, der mit einer DNA-Sequenz transformiert ist, welche fähig ist, die Herstellung des Serinprotease-Inhibitors unter Bedingungen, die für die Expression der DNA-Sequenz geeignet sind, zu lenken, und;
b) Gewinnen des Inhibitors.

2. Verfahren nach Anspruch 1, weiterhin umfassend einen Schritt, worin der Inhibitor eine aktive Tertiärstruktur einnehmen kann, wodurch dieser Serinprotease-Inhibitoraktivität erhält.

3. Verfahren nach Anspruch 1, worin die DNA-Sequenz eine synthetische DNA-Sequenz ist.

4. Verfahren nach Anspruch 1, worin die DNA-Sequenz eine natürliche DNA-Sequenz ist.

5. Verfahren nach Anspruch 1, welches weiterhin die Reinigung des Inhibitors umfaßt.

6. Verfahren nach Anspruch 5, worin die Reinigung vor dem Schritt durchgeführt wird, worin der Inhibitor eine aktive Form einnimmt.

7. Verfahren nach Anspruch 5, worin die Reinigung nach dem Schritt durchgeführt wird, worin der Inhibitor eine aktive Form einnimmt.

8. Verfahren nach Anspruch 1, worin die DNA-Sequenz auf einem Vektor getragen wird, welcher Teile der Vektoren, ausgewählt aus der Gruppe, bestehend aus pBR322 und pIQ, umfaßt.

9. Verfahren nach Anspruch 1, worin der Wirtsmikroorganismus ausgewählt ist aus der Gruppe, bestehend aus Mikroorganismen der Gattungen Escherichia, Bacillus und Saccharomyces.

10. Verfahren nach Anspruch 9, worin der Wirtsmikroorganismus Escherichia coli ist.

11. Verfahren nach Anspruch 9, worin der Wirtsmikroorganismus Bacillus subtilis ist.

12. Verfahren nach Anspruch 9, worin der Wirtsmikroorganismus Saccharomyces cerevisiae ist.

13. Verfahren nach Anspruch 4, worin die natürliche DNA-Sequenz durch ein Verfahren erhalten wird, welches die folgenden Schritte umfaßt:
a) Herstellen einer humanen cDNA-Bank aus Zellen, die fähig sind, den nativen Serinprotease-Inhibitor zu exprimieren;
b) Durchsuchen der cDNA-Bank mit mindestens einer Nukleinsäure-Sonde, welche fähig ist, an eine cDNA zu hybridisieren, die für den Inhibitor kodiert, oder mit mindestens einer Sonde, welche fähig ist, an ein durch einen Klon der cDNA-Bank exprimiertes ProteaseProtease-Inhibitorprotein zu binden;
c) Identifizieren mindestens eines Klons, welcher eine cDNA enthält, die für den Inhibitor kodiert und, wahlweise;
d) Isolieren der cDNA, die für den Inhibitor kodiert, aus dem Klon; und
e) Verbinden der cDNA, oder von Fragmenten davon, mit funktionellen Elementen, um die cDNA in einem Wirtsmikroorganismus zu halten und zu exprimieren.

14. Verfahren nach Anspruch 13, worin die Zellen humane Parotiszellen sind.

15. Verfahren nach Anspruch 4, worin die natürliche DNA-Sequenz durch ein Verfahren erhalten wird, welches die folgenden Schritte umfaßt:
a) Herstellen einer humanen, genomischen DNA-Bank;
b) Durchsuchen der genomischen DNA-Bank mit mindestens einer Nukleinsäure-Sonde, welche fähig ist, an eine genomische DNA zu hybridisieren, die für den Inhibitor kodiert, oder mit mindestens einer Sonde, welche fähig ist, an ein durch einen Klon der genomischeh DNA-Bank exprimiertes Protease-Inhibitor-Protein zu binden;
c) Identifizieren mindestens eines Klons, welcher eine genomische DNA enthält, die für den Inhibitor kodiert und wahlweise;
d) Isolieren der genomischen DNA, welche für den Inhibitor kodiert, aus dem Klon; und
e) Verbinden der genomischen DNA, oder von Fragmenten davon, mit funktionellen Elementen, um die DNA in einem Wirtsmikroorganismus zu halten und zu exprimieren.

16. Synthetische DNA-Sequenz, welche fähig ist, die mikrobielle Synthese eines Serinprotease-Inhibitors zu lenken, welcher eine einzelne, unfragmentierte Polypeptidkette umfasst und mindestens eine aktive Stelle aufweist, welche Serinprotease-Inhibitoraktivität besitzt, wobei der Inhibitor die folgende Aminosäuresequenz besitzt:

Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala.

17. DNA-Sequenz nach Anspruch 16, worin die Sequenz ausgewählt ist aus der Gruppe bestehend aus (1): 
5' AGCGG TAAAA GCTTC AAAGC TGGCG TATGC CCGCC GAAAA AATCC GCGCA GTGTC
TGCGG TACAA AAAAC CGGAA TGCCA GTCCG ACTGG CAGTG CCCGG GTAAA AAACG TTGTT
GCCCG GACAC CTGCG GCATC AAATG CCTGG ATCCG GTTGA TACCC CGAAC CCGAC TCGTC
GAAAA CCGGG TAAAT GCCCG GTAAC CTATG GCCAG TGTCT GATGC TGAAC CCGCC GAACT
TCTGC GAAAT GGACG GCCAG TGTAA ACGAG ATCTG AAATG CTGTA TGGGT ATGTG CGGCA
AATCT TGTGT TTCCC CGGTA AAAGC ATAA 3';
 oder (2) Sequenzen, welche als Folge der Degeneriertheit des genetischen Codes äquivalent zu (1) sind.

18. Rekombinanter Vektor, umfassend die DNA-Sequenz nach Anspruch 16 oder 17.

19. Wirtszelle, welche mit dem rekombinanten Vektor nach Anspruch18 transförmiert ist.

20. Isolierte DNA-Sequenz, welche für einen Serinprotease-Inhibitor, umfassendeine einzelne, unfragmentierte Polypeptidkette mit der folgenden Aminosäuresequenz, kodiert:

Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala.

21. DNA-Sequenz nach Anspruch 20, welche eine cDNA ist.

22. DNA-Sequenz nach Anspruch 20, welche eine genomische DNA. ist.

23. Rekombinanter Vektor, umfassend die DNA-Sequenz nach Anspruch 20, 21 oder 22.

24. Wirtszelle, welche mit dem rekombinanten Vektor nach Anspruch 23 transformiert ist.

25. Verfahren zur Synthese eines rekombinanten Serinprotease-Inhibitors, umfassend eine einzelne, unfragmentierte Polypeptidkette, wobei ein solches Verfahren umfaßt:
a) Herstellen einer DNA-Sequenz, welche fähig ist, einen Wirtsmikroorganismus zur Herstellung eines Polypeptids, umfassend die Aminosäuresequenz: 
R1 -Gly-Lys-Ser-Phe-Lys-Ala-Gly-Val-Cys-Pro-Pro-Lys-Lys-Ser-Ala-Gln-Cys-Leu-
R2 -Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys- R8- R3- R9-Asn-Pro-
Pro-Asn-Phe-Cys-Glu- R4-Asp-Gly-Gln-Cys-Lys-Arg-Asp-Leu-Lys-Cys-Cys- R5-Gly-
R6 -Cys-Gly-Lys-Ser-Cys-Val-Ser-Pro-Val-Lys- R7,
 zu lenken, oder eines Fragments der DNA, welches für ein Polypeptid mit mindestens einer aktiven Stelle, die Serinprotease-Inhibitoraktivität aufweist, kodiert;
worin
R₁ und R₇ gleich oder verschieden und ausgewählt sind aus der Gruppe, bestehend aus einem substituierten oder unsubstituierten Aminosäurerest oder Derivaten davon; und
R₂, R₃, R₄, R₅, R₆, R₈ und R₉ gleich oder verschieden und ausgewählt sind aus der Gruppe, bestehend aus Methionin, Valin, Alanin, Phenylalanin, Tyrosin, Tryptophan, Lysin, Glycin und Arginin,
b) Klonieren der DNA-Sequenz in einen Vektor, welcher fähig ist, in einen Wirtsmikroorganismus transferiert zu werden und darin zu replizieren, wobei ein solcher Vektor funktionelle Elemente für die DNA-Sequenz enthält;
c) Transferieren des Vektors, welcher die DNA-Sequenz und funktionellen Elemente enthält, in einen Wirtsmikroorganimsus, welcher fähig ist, den Serinprotease-Inhibitor zu exprimieren;
d) Kultivieren des Wirtsmikroorganismus unter Bedingungen, die für die Amplifizierung des Vektors und Expression des Inhibitors geeignet sind;
e) Gewinnen des Inhibitors; und
f) einen Schritt, worin der Inhibitor eine aktive Tertiärstruktur einnehmen kann, wodurch er Serinprotease-Inhibitoraktivität erhält.

26. Verfahren nach Anspruch 25, worin der Serinprotease-Inhibitor aus einer einzelnen, unfragmentierten Polypeptidkette die folgende Aminosäuresequenz: 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
 oder ein Fragment davon umfasst, wobei das Fragment mindestens eine aktive Stelle aufweist, die Serinprotease-Inhibitoraktivität besitzt.

27. DNA-Sequenz, welche fähig ist, die mikrobielle Synthese einer einzelnen, unfragmentierten polypeptidkette zu lenken, worin das Polypeptid die Aminosäuresequenz umfaßt: 
R1-Gly-Lys-Ser-Phe-Lys-Ala-Gly-Val-Cys-Pro-Pro-Lys-Lys-Ser-Ala-Gln-Cys-Leu-
R2-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys- R8- R3-R9-Asn-Pro-
Pro-Asn-Phe-Cys-Glu-R4-Asp-Gly-Gln-Cys-Lys-Arg-Asp-Leu-Lys-Cys-Cys- R5-Gly-
R6-Cys-Gly-Lys-Ser-Cys-Val-Ser-Pro-Val-Lys- R7,
 oder ein Fragment dieser DNA, welches ein Polypeptid mit mindestens einer aktiven Stelle, die Serinprotease-Inhibitoraktivität aufweist, kodiert, worin
R₁ und R₇ gleich oder verschieden und ausgewählt sind aus der Gruppe, bestehend aus einem substituierten oder unsubstituierten Aminosäurerest oder Derivaten davon; und
R₂, R₃, R₄, R₅, R₆, R₈ und R₉ gleich oder verschieden und ausgewählt sind aus der Gruppe, bestehend aus Methionin, Valin, Alanin, Phenylalanin, Tyrosin, Tryptophan, Lysin, Glycin und Arginin.

28. DNA-Sequenz nach Anspruch 27, worin der Serinprotease-Inhibitor die folgende Aminosäuresequenz 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
 oder ein Fragment davon umfasst, wobei das Fragment mindestens eine aktive Stelle besitzt, weiche Serinprotease-Inhibitoraktivität aufweist.

29. Rekombinanter Vektor, umfassend die DNA-Sequenz nach Anspruch 27 oder 28.

30. Wirtszelle, welche mit dem rekombinanten Vektor nach Anspruch 29 transformiert ist.

## Patentansprüche (Patentansprüche für folgende(n) Vertragsstaat(en): AT)

1. Verfahren zur Herstellung eines rekombinanten Serinprotease-Inhibitors, der eine einzelne, unfragmentierte Polypeptidkette umfasst, wobei der Inhibitor mindestens eine aktive Stelle aufweist, die Serinprotease-Inhibitoraktivität besitzt, wobei der Inhibitor die folgende Aminosäuresequenz aufweist: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 worin das Verfahren die folgenden Schritte umfasst:
a) Kultivieren eines Wirtsmikroorganismus, der mit einer DNA-Sequenz transformiert ist, welche fähig ist, die Herstellung des Serinprotease-Inhibitors unter Bedingungen, die für die Expression der DNA-Sequenz geeignet sind, zu lenken, und;
b) Gewinnen des Inhibitors.

2. Verfahren nach Anspruch 1, weiterhin umfassend einen Schritt, worin der Inhibitor eine aktive Tertiärstruktur einnehmen kann, wodurch dieser Serinprotease-Inhibitoraktivität erhält.

3. Verfahren nach Anspruch 1, worin die DNA-Sequenz eine synthetische DNA-Sequenz ist.

4. Verfahren nach Anspruch 1, worin die DNA-Sequenz eine natürliche DNA-Sequenz ist.

5. Verfahren nach Anspruch 1, welches weiterhin die Reinigung des Inhibitors umfasst.

6. Verfahren nach Anspruch 5, wobei die Reinigung vor dem Schritt durchgeführt wird, worin der Inhibitor eine aktive Form einnimmt.

7. Verfahren nach Anspruch 5, worin die Reinigung nach dem Schritt durchgeführt wird, worin der Inhibitor eine aktive Form einnimmt.

8. Verfahren nach Anspruch 1, worin die DNA-Sequenz auf einem Vektor getragen wird, welcher Teile der Vektoren, ausgewählt aus der Gruppe, bestehend aus pBR322 und pIQ, umfasst.

9. Verfahren nach Anspruch 1, worin der Wirtsmikroorganismus ausgewählt ist aus der Gruppe, bestehend aus Mikroorganismen der Gattungen Escherichia, Bacillus und Saccharomyces.

10. Verfahren nach Anspruch 9, worin der Wirtsmikroorganismus Escherichia coli ist.

11. Verfahren nach Anspruch 9, worin der Wirtsmikroorganismus Bacillus subtilis ist.

12. Verfahren nach Anspruch 9, worin der Wirtsmikroorganismus Saccharomyces cerevisiae ist.

13. Verfahren nach Anspruch 4, worin die natürliche DNA-Sequenz durch ein Vertahren erhalten wird, welches die folgenden Schritte umfasst:
a) Herstellen einer humanen cDNA-Bank aus Zellen, die fähig sind, den nativen Serinprotease-Inhibitor zu exprimieren;
b) Durchsuchen der cDNA-Bank mit mindestens einer Nukleinsäure-Sonde, welche fähig ist, an eine cDNA zu hybridisieren, die für den Inhibitor kodiert, oder mit mindestens einer Sonde, welche fähig ist, an ein durch einen Klon der cDNA-Bank exprimiertes Protease-Inhibitorprotein zu binden;
c) Identifizieren mindestens eines Klons, welcher eine cDNA enthält, die für den Inhibitor kodiert und, wahlweise;
d) Isolieren der cDNA, die für den Inhibitor kodiert, aus dem Klon; und
e) Verbinden der cDNA, oder von Fragmenten davon, mit funktionellen Elementen, um die cDNA in einem Wirtsmikroorganismus zu halten und zu exprimieren.

14. Verfahren nach Anspruch 13, worin die Zellen humane Parotiszellen sind.

15. Verfahren nach Anspruch 4, worin die natürliche DNA-Sequenz durch ein Verfahren erhalten wird, welches die folgenden Schritte umfasst:
a) Herstellen einer humanen, genomischen DNA-Bank;
b) Durchsuchen der genomischen DNA-Bank mit mindestens einer Nukleinsäure-Sonde, welche fähig ist, an eine genomische DNA zu hybridisieren, die für den Inhibitor kodiert, oder mit mindestens einer Sonde, welche fähig ist, an ein durch einen Klon der genomischen DNA-Bank exprimiertes Protease-Inhibitorprotein zu binden;
c) Identifizieren mindestens eines Klons, welcher eine genomische DNA enthält, die für den Inhibitor kodiert und wahlweise;
d) Isolieren der genomischen DNA, welche für den Inhibitor kodiert, aus dem Klon; und
e) Verbinden der genomischen DNA, oder von Fragmenten davon, mit funktionellen Elementen, um die DNA in einem Wirtsmikroorganismus zu halten und zu exprimieren.

16. Verfahren zur Herstellung einer synthetischen DNA-Sequenz, welche fähig ist, die mikrobielle Synthese eines Serinprotease-Inhibitors, welcher eine einzelne, unfragmentierte Polypeptidkette umfasst, zu lenken, worin der Inhibitor mindestens eine aktive Stelle aufweist, welche Serinprotease-Inhibitoraktivität besitzt, wobei der Inhibitor die folgende Aminosäuresequenz aufweist: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 umfassend das Herstellen der synthetischen DNA-Sequenz durch Polynukleotidsynthese auf eine an sich bekannte Weise.

17. Verfahren nach Anspruch 16, worin die Sequenz ausgewählt ist aus der Gruppe, bestehend aus (1): 
5' AGCGG TAAAA GCTTC AAAGC TGGCG TATGC
CCGCC GAAAA AATCC GCGCA GTGTC TGCGG
TACAA AAAAC CGGAA TGCCA GTCCG ACTGG
CAGTG CCCGG GTAAA AAACG TTGTT GCCCG
GACAC CTGCG GCATC AAATG CCTGG ATCCG
GTTGA TACCC CGAAC CCGAC TCGTC GAAAA
CCGGG TAAAT GCCCG GTAAC CTATG GCCAG
TGTCT GATGC TGAAC CCGCC GAACT TCTGC
GAAAT GGACG GCCAG TGTAA ACGAG ATCTG
AAATG CTGTA TGGGT ATGTG CGGCA AATCT
TGTGT TTCCC CGGTA AAAGC ATAA 3'
 oder (2) Sequenzen, welche als Folge der Degeneriertheit des genetischen Codes äquivalent zu (1) sind.

18. Verfahren zur Herstellung eines rekombinanten Vektors, welcher die durch das Verfahren nach Anspruch 16 oder 17 erhaltene DNA-Sequenz umfasst, umfassend das Einfügen der DNA-Sequenz in einen geeigneten Vektor.

19. Verfahren zur Herstellung einer Wirtszelle, welche mit dem durch das Verfahren nach Anspruch 18 erhaltenen rekombinanten Vektor transformiert ist, umfassend das Transformieren einer Wirtszelle mit dem Vektor.

20. Verfahren zur Herstellung einer natürlichen DNA-Sequenz, welche für einen Serinprotease-Inhibitor, umfassend eine einzelne, unfragmentierte Polypeptidkette mit der folgenden Aminosäuresequenz, kodiert; 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala.
 welches die folgenden Schritte umfasst:
a) Herstellen einer humanen cDNA-Bank aus Zellen, die fähig sind, den nativen Serinprotease-Inhibitor zu exprimieren;
b) Durchsuchen der cDNA-Bank mit mindestens einer Nukleinsäure-Sonde, welche fähig ist, an eine cDNA zu hybridisieren, die für den Inhibitor kodiert, oder mit mindestens einer Sonde, welche fähig ist, an ein durch einen Klon der cDNA-Bank exprimiertes Protease-Inhibitorprotein zu binden;
c) Identifizieren mindestens eines Klons, welcher eine cDNA enthält, die für den Inhibitor kodiert und;
d) Isolieren der cDNA, die für den Inhibitor kodiert, aus dem Klon;
oder
a) Herstellen einer humanen, genomischen DNA-Bank;
b) Durchsuchen der genomischen DNA-Bank mit mindestens einer Nukleinsäure-Sonde, welche fähig ist, an eine genomische DNA zu hybridisieren, die für den Inhibitor kodiert, oder mit mindestens einer Sonde, welche fähig ist, an ein durch einen Klon der genomischen DNA-Bank exprimiertes Protease-Inhibitorprotein zu binden;
c) Identifizieren mindestens eines Klons, welcher eine genomische DNA enthält, die für den Inhibitor kodiert und;
d) Isolieren der genomischen DNA, welche für den Inhibitor kodiert, aus dem Klon.

21. Verfahren nach Anspruch 20, worin die DNA-Sequenz eine cDNA ist.

22. Verfahren nach Anspruch 20, worin die DNA-Sequenz eine genomische DNA ist.

23. Verfahren zur Herstellung eines rekombinanten Vektors, welcher die durch das Verfahren nach Anspruch 20, 21 oder 22 erhaltene DNA-Sequenz umfasst, umfassend das Einfügen der DNA-Sequenzen in einen geeigneten Vektor.

24. Verfahren zur Herstellung einer Wirtszelle, welche mit dem durch das Verfahren nach Anspruch 23 erhaltenen rekombinanten Vektor transformiert ist, umfassend das Transformieren einer Wirtszelle mit dem Vektor.

25. Verfahren zur Synthese eines rekombinanten Serinprotease-Inhibitors, umfassend eine einzelne, unfragmentierte Polypeptidkette, wobei ein solches Verfahren umfasst:
a) Herstellen einer DNA-Sequenz, welche fähig ist, einen Wirtsmikroorganismus zur Herstellung eines Polypeptids, umfassend die Aminosäuresequenz: 
R1-Gly-Lys-Ser-Phe-Lys-Ala-Gly-Val-Cys-Pro-
Pro-Lys-Lys-Ser-Ala-Gln-Cys-Leu-R2-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-R8-R3-R9-Asn-Pro-Pro-
Asn-Phe-Cys-Glu-R4-Asp-Gly-Gln-Cys-Lys-Arg-
Asp-Leu-Lys-Cys-Cys-R5-Gly-R6-Cys-Gly-Lys-
Ser-Cys-Val-Ser-Pro-Val-R7,
 zu lenken, oder eines Fragments der DNA, welches für ein Polypeptid mit mindestens einer aktiven Stelle, die Serinprotease-Inhibitoraktivität aufweist, kodiert;
worin
R₁ und R₇ gleich oder verschieden und ausgewählt sind aus der Gruppe, bestehend aus einem substituierten oder unsubstituierten Aminosäurerest oder Derivaten davon; und
R₂, R₃, R₄, R₅, R₆, R₈ und R₉ gleich oder verschieden und ausgewählt sind aus der Gruppe, bestehend aus Methionin, Valin, Alanin, Phenylalanin, Tyrosin, Tryptophan, Lysin, Glycin und Arginin,
b) Klonieren der DNA-Sequenz in einen Vektor, welcher fähig ist, in einen Wirtsmikroorganismus transferiert zu werden und darin zu replizieren, wobei ein solcher Vektor funktionelle Elemente für die DNA-Sequenz enthält;
c) Transferieren des Vektors, welcher die DNA-Sequenz und funktionellen Elemente enthält, in einem Wirtsmikroorganimsus, welcher fähig ist, den Serinprotease-Inhibitor zu exprimieren;
d) Kultivieren des Wirtsmikroorganismus unter Bedingungen, die für die Amplifizierung des Vektors und Expression des Inhibitors geeignet sind;
e) Gewinnen des Inhibitors; und
f) einen Schritt, worin der Inhibitor eine aktive Tertiärstruktur einnehmen kann, wodurch er Serinprotease-Inhibitoraktivität erhält.

26. Verfahren nach Anspruch 25, worin der Serinprotease-Inhibitor aus einer einzelnen, unfragmentierten Polypeptidkette die folgende Aminosäuresequenz: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 oder ein Fragment davon umfasst, wobei das Fragment mindestens eine aktive Stelle aufweist, die Serinprotease-Inhibitoraktivität besitzt.

27. Verfahren zur Herstellung einer DNA-Sequenz, welche fähig ist, die mikrobielle Synthese einer einzelnen, unfragmentierten Polypeptidkette zu lenken, worin das Polypeptid die Aminosäuresequenz umfasst: 
R1-Gly-Lys-Ser-Phe-Lys-Ala-Gly-Val-Cys-Pro-
Pro-Lys-Lys-Ser-Ala-Gln-Cys-Leu-R2-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-R8-R3-R9-Asn-Pro-Pro-
Asn-Phe-Cys-Glu-R4-Asp-Gly-Gln-Cys-Lys-Arg-
Asp-Leu-Lys-Cys-Cys-R5-Gly-R6-Cys-Gly-Lys-
Ser-Cys-Val-Ser-Pro-Val-Lys-R7,
 oder eines Fragments dieser DNA, welches ein Polypeptid mit mindestens einer aktiven Stelle, die Serinprotease-Inhibitoraktivität aufweist, kodiert, worin
R₁ und R₇ gleich oder verschieden und ausgewählt sind aus der Gruppe, bestehend aus einem substituierten oder unsubstituierten Aminosäurerest oder Derivaten davon; und
R₂, R₃, R₄, R₅, R₆, R₈ und R₉ gleich oder verschieden und ausgewählt sind aus der Gruppe, bestehend aus Methionin, Valin, Alanin, Phenylalanin, Tyrosin, Tryptophan, Lysin, Glycin und Arginin, umfassend das Herstellen der DNA-Sequenz auf eine an sich bekannte Weise.

28. Verfahren nach Anspruch 27, worin der Serinprotease-Inhibitor die folgende Aminosäuresequenz: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 oder ein Fragment davon umfasst, wobei das Fragment mindestens eine aktive Stelle besitzt, welche Serinprotease-Inhibitoraktivität aufweist.

29. Verfahren zur Herstellung eines rekombinanten Vektors, welcher die durch das Verfahren nach Anspruch 27 oder 28 erhaltene DNA-Sequenz umfasst, umfassend das Einfügen der DNA-Sequenz in einen geeigneten Vektor.

30. Verfahren zur Herstellung einer Wirtszelle, welche mit dem durch das Verfahren nach Anspruch 29 erhaltenen rekombinanten Vektor transformiert ist, umfassend das Transformieren einer Wirtszelle mit dem Vektor.

## Revendications (Revendications pour l'(les) Etat(s) contractant(s) suivant(s): BE, CH, LI, DE, FR, GB, IT, LU, NL, SE)

1. Procédé pour la production d'un inhibiteur recombinant de sérine protéase, comprenant une seule chaîne polypeptidique non fragmentée et comportant au moins un site actif possédant une activité d'inhibiteur de sérine protéase, ledit inhibiteur ayant la séquence d'acides aminés suivante: 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
 ledit procédé comprenant les étapes suivantes:
a) culture d'un micro-organisme hôte transformé par une séquence d'ADN capable d'induire la production de l'inhibiteur de sérine protéase dans des conditions appropriées à l'expression de la séquence d'ADN, et
b) récolte de l'inhibiteur.

2. Procédé selon la revendication 1, comprenant en outre l'étape consistant à permettre à l'inhibiteur d'adopter une structure tertiaire active grâce à laquelle il possède une activité d'inhibiteur de sérine protéase.

3. Procédé selon la revendication 1, dans lequel ladite séquence d'ADN est une séquence d'ADN synthétique.

4. Procédé selon la revendication 1, dans lequel ladite séquence d'ADN est une séquence d'ADN naturel.

5. Procédé selon la revendication 1, qui comprend en outre la purification de l'inhibiteur.

6. Procédé selon la revendication 5, dans lequel ladite purification est effectuée avant l'étape consistant à permettre audit inhibiteur d'adopter une forme active.

7. Procédé selon la revendication 5, dans lequel la purification est effectuée après l'étape consistant à permettre audit inhibiteur d'adopter une forme active.

8. Procédé selon la revendication 1, dans lequel la séquence d'ADN est portée sur un vecteur comprenant des parties des vecteurs choisis parmi pBR322 et pIQ.

9. Procédé selon la revendication 1, dans lequel le micro-organisme hôte est choisi parmi des micro-organismes appartenant aux genres *Escherichia, Bacillus* et *Saccharomyces.*

10. Procédé selon la revendication 9, dans lequel le micro-organisme hôte est *Escherichia coli*.

11. Procédé selon la revendication 9, dans lequel le micro-organisme hôte est *Bacillus szrbtilis.*

12. Procédé selon la revendication 9, dans lequel le micro-organisme hôte est *Saccharomyces cerevisiae.*

13. Procédé selon la revendication 4, dans lequel la séquence d'ADN naturel est obtenue par un procédé comprenant les étapes suivantes:
a) préparation d'une banque de fragments d'ADNc humain provenant de cellules capables d'exprimer l'inhibiteur natif de sérine protéase;
b) sondage de la banque d'ADNc à l'aide d'au moins une sonde d'acide nucléique capable d'hybridation avec un ADNc codant pour l'inhibiteur ou à l'aide d'au moins une sonde capable de liaison à une protéine inhibitrice de protéase, exprimée par un clone de la banque d'ADNc;
c) identification d'au moins un clone contenant un ADNc codant pour l'inhibiteur, et, éventuellement
d) isolement de l'ADNc codant pour l'inhibiteur provenant du clone; et
e) liaison de l'ADNc, ou de fragments de celui-ci, à des éléments fonctionnels pour maintenir et exprimer l'ADNc dans un micro-organisme hôte.

14. Procédé selon la revendication 13, dans lequel les cellules sont des cellules parotidiennes humaines.

15. Procédé selon la revendication 4, dans lequel ladite séquence d'ADN naturelle est obtenue par un procédé comprenant les étapes suivantes:
a) préparation d'une banque de fragments d'ADN génomique humain;
b) sondage de la banque de fragments d'ADN génomique à l'aide d'au moins une sonde d'acide nucléique capable d'hybridation avec un ADN génomique codant pour l'inhibiteur ou à l'aide d'au moins une sonde capable de liaison à une protéine inhibitrice de protéase, exprimée par un clone de la banque d'ADN génomique;
c) identification d'au moins un clone contenant un ADN génomique codant pour l'inhibiteur et, éventuellement,
d) isolement de l'ADN génomique codant pour l'inhibiteur provenant du clone; et
e) liaison de l'ADN génomique, ou de fragments de celui-ci, à des éléments fonctionnels pour le maintien et l'expression de l'ADN dans un micro-organisme hôte.

16. Séquence d'ADN synthétique capable de diriger la synthèse microbienne d'un inhibiteur de sérine protéase comprenant une seule chaîne polypeptidique non fragmentée, comportant au moins un site actif possédant une activité d'inhibiteur de sérine protéase, ledit inhibiteur ayant la séquence d' acides aminés suivantes :

Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala.

17. Séquence d'ADN selon la revendication 16, ladite séquence étant choisie dans le groupe constitué par (1): 
5'    AGCGG TAAAA GCTTC AAAGC TGGCG TATGC CCGCC GAAAA AATCC GCGCA GTGTC
TGCGG TACAA AAAAC CGGAA TGCCA GTCCG ACTGG CAGTG CCCGG GTAAA AAACG TTGTT
GCCCG GACAC CTGCG GCATC AAATG CCTGG ATCCG GTTGA TACCC CGAAC CCGAC TCGTC
GAAAA CCGGG TAAAT GCCCG GTAAC CTATG GCCAG TGTCT GATGC TGAAC CCGCC GAACT
TCTGC GAAAT GGACG GCCAG TGTAA ACGAG ATCTG AAATG CTGTA TGGGT ATGTG CGGCA
AATCT TGTGT TTCCC CGGTA AAAGC ATAA 3';
 et (2) des séquences qui sont équivalentes à la séquence (1), par suite de la dégénérescence du code génétique.

18. Vecteur recombinant comprenant la séquence d'ADN de la revendication 16 ou 17.

19. Cellule hôte transformée par le vecteur recombinant de la revendication 18.

20. Séquence d'ADN isolée qui code pour un inhibiteur de sérine protéase comprenant une seule chaîne polypeptidique non fragmentée ayant la séquence acides aminés suivante:

Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala.

21. Séquence d'ADN selon la revendication 20, qui est un ADNc.

22. Séquence d'ADN selon la revendication 20, qui est un ADN génomique.

23. Vecteur recombinant comprenant la séquence d'ADN de la revendication 20, 21 ou 22.

24. Cellule hôte transformée par le vecteur recombinant de la revendication 23.

25. Procédé pour la synthèse d'un inhibiteur recombinant de sérine protéase comprenant une seule chaîne polypeptidique non fragmentée, lequel procédé comprenant:
a) la préparation d'une séquence d'ADN capable d'induire un micro-organisme hôte à produire un polypeptide comprenant la séquence d'acides aminés : 
R1 -Gly-Lys-Ser-Phe-Lys-Ala-Gly-Val-Cys-Pro-Pro-Lys-Lys-Ser-Ala-Gln-Cys-Leu-
R2 -Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys- R8- R3- R9-Asn-Pro-
Pro-Asn-Phe-Cys-Glu- R4-Asp-Gly-Gln-Cys-Lys-Arg-Asp-Leu-Lys-Cys-Cys- R5-Gly-
R6 -Cys-Gly-Lys-Ser-Cys-Val-Ser-Pro-Val-Lys- R7,
 ou un fragment de cet ADN codant pour un polypeptide comportant au moins un site actif possédant une activité d'inhibiteur de sérine protéase, séquence dans laquelle
R₁ et R₇ sont identiques ou différents et sont choisis parmi des acides aminés substitués ou non substitués et leurs dérivés; et
R₂, R₃, R₄, R₅, R₆, R₈ et R₉ sont identiques ou différents et sont choisis parmi les méthionine, valine, alanine, phénylalanine, tyrosiné, tryptophane, lysine, glyciné et arginine;
b) le clonage de la séquence d'ADN dans un vecteur capable d'être transféré dans et de se répliquer dans un micro-organisme hôte, ce vecteur contenant des éléments fonctionnels pour la séquence d'ADN;
c) le transfert du vecteur contenant la séquence d'ADN et les éléments fonctionnels dans un micro-organisme hôte capable d'exprimer l'inhibiteur de sérine protéase;
d) la culture du micro-organisme hôte dans des conditions appropriées à l'amplification du vecteur et à l'expression de l'inhibiteur;
e) la récolte de l'inhibiteur; et
f) une étape consistant à permettre à l'inhibiteur d'adopter une structure tertiaire active grâce à laquelle il possède une activité d'inhibiteur de sérine protéase.

26. Procédé selon la revendication 25, dans lequel ledit inhibiteur de sérine protéase, comprenant une seule chaine polypeptidique non fragmentée, comprend la séquence d'acides aminés : 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
 ou un fragment de celle-ci, ledit fragment comportant au moins un site actif possédant une activité d'inhibiteur de sérine protéase.

27. Séquence d'ADN capable d'induire la synthèse microbienne d'une chaîne polypeptidique unique non fragmentée, **caractérisée en ce que** ledit polypeptide comprend la séquence d' acides aminés : 
R1-Gly-Lys-Ser-Phe-Lys-Ala-Gly-Val-Cys-Pro-Pro-Lys-Lys-Ser-Ala-Gln-Cys-Leu-
R2-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys- R8- R3-R9-Asn-Pro-
Pro-Asn-Phe-Cys-Glu-R4-Asp-Gly-Gln-Cys-Lys-Arg-Asp-Leu-Lys-Cys-Cys- R5-Gly-
R6-Cys-Gly-Lys-Ser-Cys-Val-Ser-Pro-Val-Lys- R7,
 ou un fragment de cet ADN codant pour un polypeptide comportant au moins un site actif possédant une activité d'inhibiteur de sérine protéase;
séquence dans laquelle
R₁ et R₇ sont identiques ou différents et sont choisis parmi des acides aminés substitués ou non substitués et leurs dérivés; et
R₂, R₃, R₄, R₅, R₆, R₈ et R₉ sont identiques ou différents et sont choisis parmi les méthionine, valine, alanine, phénylalanine, tyrosine, tryptophane, lysine, glycine et arginine.

28. Séquence d'ADN selon la revendication 27, **caractérisée en ce que** ledit inhibiteur de sérine protéase comprend la séquence d' acides aminés : 
Ser-Gly-Lys-Ser-Phe-Lys-Ala-Gly-Val-Cys-Pro-Pro-Lys-Lys-Ser-Ala-Gln-Cys-Leu-
Arg-Tyr-Lys-Lys-Pro-Glu-Cys-Gln-Ser-Asp-Trp-Gln-Cys-Pro-Gly-Lys-Lys-Arg-Cys-
Cys-Pro-Asp-Thr-Cys-Gly-Ile-Lys-Cys-Leu-Asp-Pro-Val-Asp-Thr-Pro-Asn-Pro-Thr-
Arg-Arg-Lys-Pro-Gly-Lys-Cys-Pro-Val-Thr-Tyr-Gly-Gln-Cys-Leu-Met-Leu-Asn-Pro-
Pro-Asn-Phe-Cys-Glu-Met-Asp-Gly-Gln-Cys-Lys-Arg-Asp-Leu-Lys-Cys-Cys-Met-Gly-
Met-Cys-Gly-Lys-Ser-Cys-Val-Ser-Pro-Val-Lys-Ala,
ou un fragment de celle-ci,ledit fragment comportant au moins un site actif possédant une activité d'inhibiteur de sérine protéase.

29. Vecteur recombinant comprenant la séquence d'ADN de la revendication 27 ou 28.

30. Cellule hôte transformée par le vecteur recombinant de la revendication 29.

## Revendications (Revendications pour l'(les) Etat(s) contractant(s) suivant(s): AT)

1. Procédé pour la production d'un inhibiteur recombinant de sérine protéase, comprenant une seule chaîne polypeptidique non fragmentée, ledit inhibiteur comportant au moins un site actif possédant une activité d'inhibiteur de sérine protéase, ledit inhibiteur comportant la séquence d'acides aminés suivante : 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala.
 ledit procédé comprenant les étapes suivantes:
a) culture d'un micro-organisme hôte transformé par une séquence d'ADN capable d'induire la production de l'inhibiteur de sérine protéase dans des conditions appropriées à l'expression de la séquence d'ADN, et
b) récolte de l'inhibiteur.

2. Procédé selon la revendication 1, comprenant en outre l'étape consistant à permettre à l'inhibiteur d'adopter une structure tertiaire active grâce à laquelle il possède une activité d'inhibiteur de sérine protéase.

3. Procédé selon la revendication 1, dans lequel ladite séquence d'ADN est une séquence d'ADN synthétique.

4. Procédé selon la revendication 1, dans lequel ladite séquence d'ADN est une séquence d'ADN naturel.

5. Procédé selon la revendication 1, qui comprend en outre la purification de l'inhibiteur.

6. Procédé selon la revendication 5, dans lequel ladite purification est effectuée avant l'étape consistant à permettre audit inhibiteur d'adopter une forme active.

7. Procédé selon la revendication 5, dans lequel la purification est effectuée après l'étape consistant à permettre audit inhibiteur d'adopter une forme active.

8. Procédé selon la revendication 1, dans lequel la séquence d'ADN est portée sur un vecteur comprenant des parties des vecteurs choisis parmi pBR322 et pIQ.

9. Procédé selon la revendication 1, dans lequel le micro-organisme hôte est choisi parmi des micro-organismes appartenant aux genres *Escherichia, Bacillus* et *Saccharomyces.*

10. Procédé selon la revendication 9, dans lequel le micro-organisme hôte est *Escherichia coli.*

11. Procédé selon la revendication 9, dans lequel le micro-organisme hôte est *Bacillus subtilis.*

12. Procédé selon la revendication 9, dans lequel le micro-organisme hôte est *Saccharomyces cerevisiae*.

13. Procédé selon la revendication 4, dans lequel la séquence d'ADN naturel est obtenue par un procédé comprenant les étapes suivantes:
a) préparation d'une banque de fragments d'ADNc humain provenant de cellules capables d'exprimer l'inhibiteur natif de sérine protéase;
b) sondage de la banque d'ADNc à l'aide d'au moins une sonde d'acide nucléique capable d'hybridation avec un ADNc codant pour l'inhibiteur ou à l'aide d'au moins une sonde capable de liaison à une protéine inhibitrice de protéase, exprimée par un clone de la banque d'ADNc;
c) identification d'au moins un clone contenant un ADNc codant pour l'inhibiteur, et, éventuellement
d) isolement de l'ADNc codant pour l'inhibiteur provenant du clone; et
e) liaison de l'ADNc, ou de fragments de celui-ci, à des éléments fonctionnels pour maintenir et exprimer l'ADNc dans un micro-organisme hôte.

14. Procédé selon la revendication 13, dans lequel les cellules sont des cellules parotidiennes humaines.

15. Procédé selon la revendication 4, dans lequel ladite séquence d'ADN naturelle est obtenue par un procédé comprenant les étapes suivantes:
a) préparation d'une banque de fragments d'ADN génomique humain;
b) sondage de la banque de fragments d'ADN génomique à l'aide d'au moins une sonde d'acide nucléique capable d'hybridation avec un ADN génomique codant pour l'inhibiteur ou à l'aide d'au moins une sonde capable de liaison à une protéine inhibitrice de protéase, exprimée par un clone de la banque d'ADN génomique;
c) identification d'au moins un clone contenant un ADN génomique codant pour l'inhibiteur, et, éventuellement,
d) isolement de l'ADNc génomique codant pour l'inhibiteur provenant du clone; et
e) liaison de l'ADNc, génomique, ou de fragments de celui-ci, à des éléments fonctionnels pour le maintenir et l'expression de l'ADN dans un micro-organisme hôte.

16. Procédé pour la production d'une séquence d'ADN synthétique capable d'induire la synthèse microbienne d'un inhibiteur de sérine protéase comprenant une seule chaîne polypeptidique non fragmentée, ledit inhibiteur comportant au moins un site actif possédant une activité d'inhibiteur de sérine protéase, ledit inhibiteur
ayant la séquence d'acides aminés suivantes: 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 comprenant la préparation de ladite séquence d'ADN synthétique par synthèse de polynucléotides, d'une façon connue en soi.

17. Procédé selon la revendication 16, dans lequel ladite séquence est choisie dans le groupe constitué par (1): 
5'	AGCGG TAAAA GCTTC AAAGC TGGCG TATGC
CCGCC GAAAA AATCC GCGCA GTGTC TGCGG
TACAA AAAAC CGGAA TGCCA GTCCG ACTGG
CAGTG CCCGG GTAAA AAACG TTGTT GCCCG
GACAC CTGCG GCATC AAATG CCTGG ATCCG
GTTGA TACCC CGAAC CCGAC TCGTC GAAAA
CCGGG TAAAT GCCCG GTAAC CTATG GCCAG
TGTCT GATGC TGAAC CCGCC GAACT TCTGC
GAAAT GGACG GCCAG TGTAA ACGAG ATCTG
AAATG CTGTA TGGGT ATGTG CGGCA AATCT
TGTGT TTCCC CGGTA AAAGC ATAA 3'
 et (2) des séquences qui sont équivalentes à la séquence (1), par suite de la dégénérescence du code génétique.

18. Procédé pour la production d'un vecteur recombinant qui comprend la séquence d'ADN obtenue par le procédé de la revendication 16 ou 17, comprenant l'insertion de ladite séquence d'ADN dans un vecteur approprié.

19. Procédé pour la production d'une cellule hôte transformée par le vecteur recombinant obtenu par le procédé de la revendication 18, comprenant la transformation d'une cellule hôte par ledit vecteur.

20. Procédé pour la production d'une séquence d'ADN naturel qui code pour un inhibiteur de sérine protéase comprenant une seule chaîne polypeptidique non fragmentée ayant la séquence d' acides aminés suivante 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala.
 comprenant les étapes suivantes:
a) préparation d'une banque de fragments d'ADNc humain à partir de cellules capables d'exprimer l'inhibiteur natif de sérine protéase;
b) sondage de la banque d'ADNc à l'aide d'au moins une sonde d'acide nucléique capable d'hybridation avec un ADNc codant pour l'inhibiteur, ou à l'aide d'au moins une sonde capable de liaison à une protéine inhibitrice de protéase, exprimée par un clone de la banque d'ADNc;
c) identification d'au moins un clone contenant un ADNc codant pour l'inhibiteur, et
d) isolement de l'ADNc codant pour l'inhibiteur à partir du clone; ou
a) préparation d'une banque de fragments d'ADN génomique humain;
b) sondage de la banque de fragments d'ADN génomique à l'aide d'au moins une sonde d'acide nucléique capable d'hybridation avec un ADN génomique codant pour l'inhibiteur, ou à l'aide d'au moins une sonde capable de liaison à une protéine inhibitrice de protéase exprimée par un clone de la banque d'ADN génomique;
c) identification d'au moins un clone contenant un ADN génomique codant pour l'inhibiteur, et
d) isolement de l'ADN génomique codant pour l'inhibiteur à partir du clone.

21. Procédé selon la revendication 20, dans lequel la séquence d'ADN est un ADNc.

22. Procédé selon la revendication 20, dans lequel la séquence d'ADN est un ADN génomique.

23. Procédé pour la production d'un vecteur recombinant qui comprend la séquence d'ADN obtenue par le procédé des revendications 20, 21 ou 22, comprenant l'insertion desdites séquences d'ADN dans un vecteur approprié.

24. Procédé pour la production d'une cellule hôte transformée par le vecteur recombinant obtenu par le procédé de la revendication 23, comprenant la transformation d'une cellule hôte par ledit vecteur.

25. Procédé pour la synthèse d'un inhibiteur recombinant de sérine protéase comprenant une seule chaîne polypeptidique non fragmentée, lequel procédé comprenant:
a) la préparation d'une séquence d'ADN capable d'induire un micro-organisme hôte à produire un polypeptide comprenant la séquence d'acides aminés : 
R1-Gly-Lys-Ser-Phe-Lys-Ala-Gly-Val-Cys-Pro-
Pro-Lys-Lys-Ser-Ala-Gln-Cys-Leu-R2-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-R8-R3-R9-Asn-Pro-Pro-
Asn-Phe-Cys-Glu-R4-Asp-Gly-Gln-Cys-Lys-Arg-
Asp-Leu-Lys-Cys-Cys-R5-Gly-R6-Cys-Gly-Lys-
Ser-Cys-Val-Ser-Pro-Val-R7,
 ou un fragment de cet ADN codant pour un polypeptide comportant au moins un site actif possédant une activité d'inhibiteur de sérine protéase, séquence dans laquelle
R₁ et R₇ sont identiques ou différents et sont choisis parmi des acides aminés substitués ou non substitués et leurs dérivés; et
R₂, R₃, R₄, R₅, R₆, R₈ et R₉ sont identiques ou différents et sont choisis parmi les méthionine, valine, alanine, phénylalanine, tyrosine, tryptophane, lysine, glycine et arginine;
b) le clonage de la séquence d'ADN dans un vecteur capable d'être transféré dans et de se répliquer dans un micro-organisme hôte, ce vecteur contenant des éléments fonctionnels pour la séquence d'ADN;
c) le transfert du vecteur contenant la séquence d'ADN et des éléments fonctionnels dans un micro-organisme hôte capable d'exprimer l'inhibiteur de sérine protéase;
d) la culture du micro-organisme hôte dans des conditions appropriées à l'amplification du vecteur et à l'expression de l'inhibiteur;
e) la récolte de l'inhibiteur; et
f) une étape consistant à permettre à l'inhibiteur d'adopter une structure tertiaire active grâce à laquelle il possède une activité d'inhibiteur de sérine protéase.

26. Procédé selon la revendication 25, dans lequel ledit inhibiteur de sérine protéase, comprenant une seule chaîne polypeptidique non fragmentée, comprend la séquence d'acides aminés : 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 ou un fragment de celle-ci, ledit fragment comportant au moins un site actif possédant une activité d'inhibiteur de sérine protéase.

27. Procédé pour la production d'une séquence d'ADN capable d'induire la synthèse microbienne d'une chagne polypeptidique unique non fragmentée, dans lequel ledit polypeptide comprend la séquence d'acides aminés : 
R1-Gly-Lys-Ser-Phe-Lys-Ala-Gly-Val-Cys-Pro-
Pro-Lys-Lys-Ser-Ala-Gln-Cys-Leu-R2-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-R8-R3-R9-Asn-Pro-Pro-
Asn-Phe-Cys-Glu-R4-Asp-Gly-Gln-Cys-Lys-Arg-
Asp-Leu-Lys-Cys-Cys-R5-Gly-R6-Cys-Gly-Lys-
Ser-Cys-Val-Ser-Pro-Val-Lys-R7,
 ou un fragment de cet ADN codant pour un polypeptide comportant au moins un site actif possédant une activité d'inhibiteur de sérine protéase;
séquence dans laquelle
R₁ et R₇ sont identiques ou différents et sont choisis parmi des acides aminés substitués ou non substitués et leurs dérivés; et
R₂, R₃, R₄, R₅, R₆, R₈ et R₉ sont identiques ou différents et sont choisis parmi les méthionine, valine, alanine, phénylalanine, tyrosine, tryptophane, lysine, glycine et arginine,
comprenant la préparation de ladite séquence d'ADN d'une façon connue en soi.

28. Procédé selon la revendication 27, dans lequel ledit inhibiteur de sérine protéase comprend la séquence d'acides aminés : 
Ser-Gly-Lys-Ser-Phe-Lys-Ala-Gly-Val-Cys-Pro-
Pro-Lys-Lys-Ser-Ala-Gln-Cys-Leu-Arg-Tyr-Lys-
Lys-Pro-Glu-Cys-Gln-Ser-Asp-Trp-Gln-Cys-Pro-
Gly-Lys-Lys-Arg-Cys-Cys-Pro-Asp-Thr-Cys-Gly-
Ile-Lys-Cys-Leu-Asp-Pro-Val-Asp-Thr-Pro-Asn-
Pro-Thr-Arg-Arg-Lys-Pro-Gly-Lys-Cys-Pro-Val-
Thr-Tyr-Gly-Gln-Cys-Leu-Met-Leu-Asn-Pro-Pro-
Asn-Phe-Cys-Glu-Met-Asp-Gly-Gln-Cys-Lys-Arg-
Asp-Leu-Lys-Cys-Cys-Met-Gly-Met-Cys-Gly-Lys-
Ser-Cys-Val-Ser-Pro-Val-Lys-Ala,
 ou un fragment de celle-ci, ledit fragment comportant au moins un site actif possédant une activité d'inhibiteur de sérine protéase.

29. Procédé pour la production d'un vecteur recombinant qui comprend la séquence d'ADN obtenue par le procédé de la revendication 27 ou 28, comprenant l'insertion de ladite séquence d'ADN dans un vecteur approprié.

30. Procédé pour la production d'une cellule hôte transformée par le vecteur recombinant obtenu par le procédé de la revendication 29, comprenant la transformation d'une cellule hôte par ledit vecteur.
